# EUROPÄISCHE PATENTSCHRIFT

(11) **EP 4 076 140 B1**
(45) Veröffentlichungstag und Bekanntmachung des Hinweises auf die Patenterteilung: **21.08.2024**
(21) Anmeldenummer: 20817207.2
(22) Anmeldetag: 23.11.2020
(51) Int. Cl.: A61B 1/01

(54) **OPHTHALMOSKOP ZUR UNTERSUCHUNG VON AUGEN**
OPHTHALMOSCOPE FOR EXAMINING EYES
OPHTALMOSCOPE DESTINÉE À L'EXAMEN DES YEUX

(30) Priorität: 19.12.2019 AT 602822019
(43) Veröffentlichungstag der Anmeldung: 26.10.2022
(73) Patentinhaber: Occyo GmbH, 6020 Innsbruck (AT)
(72) Erfinder: HAUSMANN, Ulrich, 6020 Innsbruck (AT)
(74) Vertreter: Torggler & Hofmann Patentanwälte - Innsbruck
(86) Internationale Anmeldenummer: PCT/AT2020/060415
(87) Internationale Veröffentlichungsnummer: WO 2021/119684

(56) Entgegenhaltungen:
- EP-A1- 2 728 545
- EP-A1- 3 272 395
- EP-A1- 3 424 406
- EP-A1- 3 539 460
- WO-A1-2019/189222
- JP-A- H0 824 223
- RU-C2- 2 440 802
- US-A1- 2006 146 283
- US-A1- 2011 286 102
- US-A1- 2012 069 299
- US-B2- 6 577 387
- US-B2- 7 621 638

## Beschreibung

Die Erfindung betrifft ein Ophthalmoskop zur Untersuchung einer sichtbaren Oberfläche von Augen umfassend ein Gehäuse, wenigstens eine Vorrichtung zur Umwandlung von Licht in ein elektrisches Signal in Form eines planaren Chips und wenigstens ein Objektiv, wobei das wenigstens eine Objektiv wenigstens eine Linse und/oder wenigstens einen Spiegel umfasst. Weiters betrifft die Erfindung ein solches Ophthalmoskop mit einer Spaltlampe sowie eine Verwendung eines solchen Ophthalmoskops am menschlichen Auge. Des Weiteren betrifft die Erfindung ein Verfahren zur Untersuchung von Augen durch ein solches Ophthalmoskop sowie ein Ophthalmoskop mit einem Computerprogrammprodukt.

Handelsübliche Ophthalmoskope, welche Augen meist unter der Verwendung einer Spaltlampe untersuchen, können nur auf eine Ebene fokussieren. Da eine komplexe Augengeometrie von einer Ebene abweicht, ist es nur möglich, eine Ringzone des Auges scharf auf die Vorrichtung zur Umwandlung insbesondere bei einer Vergrößerung des Auges um das Zehnfache abzubilden, wobei die restliche Oberfläche des Auges unscharf durch handelsübliche Ophthalmoskope abgebildet wird. Diese Ophthalmoskope sind ungeeignet für eine effiziente und effektive Untersuchung von Augen und da für eine vollständige scharfe Abbildung des Auges eine Vielzahl von Abbildungen notwendig ist, welche jeweils nur einen Bereich des Auges scharf abbilden, müsste das Auge über einen Zeitraum sämtlicher Abbildungen exakt gleich positioniert sein, wobei dies einem praktisch nicht umsetzbaren Ideal entspricht. Folglich sind eine Reproduzierbarkeit, eine einwandfreie Analyse sowie ein Vergleich durch standardisierte Abbildungen schlicht nicht möglich. Die benötigte Ringzone des Auges und selbige Ringzone des Auges über mehrere Aufnahmen, gegebenenfalls bei unterschiedlichen Augen zu erfassen, wird durch handelsübliche Ophthalmoskope erschwert.

Ein System zur Inspektion einer ophthalmischen Linse ist bereits aus der Schrift US 6,577,387 B2 bekannt, in welcher Änderungen in einer Intensität des Lichtes einer Abbildung auf einer Vorrichtung zur Umwandlung von Licht - hervorgerufen durch Änderungen einer Dicke der ophthalmischen Linse - analysiert werden. Die Änderungen in der Dicke der ophthalmischen Linse werden durch kosmetische Fehler, Unregelmäßigkeiten oder durch ein Design der ophthalmischen Linse verursacht. Eine Abbildungslinsenanordnung in Form eines Objektivs kann eine gekrümmte Brennfläche aufweisen, um hinsichtlich der Krümmung der ophthalmischen Linse eine Verbesserung des Fokus zu generieren.

Nachteilig am Stand der Technik ist, dass die Inspektion der ophthalmischen Linsen unter der Verwendung einer Absorptionstechnik unter der Rücksichtnahme des Lambert-Beer'schen bei ophthalmischen Linsen erfolgt und nicht für die Untersuchung von Augen mit einer komplexen konvexen Geometrie, welche von einer ophthalmischen Linse verschieden ist, herangezogen werden kann. Zudem wird in der Absorptionstechnik Licht mit Wellenlängen im Ultraviolettbereich, im Infrarotbereich oder Laserlicht mit monochromatischem Licht genützt, welche bei den für die Absorptionstechnik geforderten Leistungen für die Untersuchung von Augen aus gesundheitlichen Gründen ungeeignet sind. Für sichtbares Licht wären zusätzliche optische Elemente zur Manipulation von Licht erforderlich, um die Inspektion der ophthalmischen Linsen mit der Absorptionstechnik untersuchen zu können. Des Weiteren ist nicht klar, wie eine Standardisierung der Abbildungen der ophthalmischen Linsen für einen Vergleich anhand spezifischer Parameter erfolgen könnte, um eine Vielzahl von Abbildungen für eine Analyse heranziehen zu können. Weiters ist nachteilig, dass das Licht zuerst die ophthalmische Linse passieren muss, um anhand eines Absorptionsspektrum auf die Eigenschaften der ophthalmischen Linse rückschließen zu können.

Weitere Ophthalmoskope - insbesondere zur Abbildung konkaver Fundus-Geometrien - gehen aus den Schriften WO 2019/189222A1, US 2003/218755 A1, EP 2 728 545 A1, EP 3 539 460 A1, US 2006/146283 A1, JP H0824223 A, DE 10 2018 218 107 A1, US 2012/069299 A1 sowie US 7,621,638 B2 hervor.

Die objektiv technische Aufgabe der vorliegenden Erfindung besteht daher darin, ein gegenüber dem Stand der Technik verbessertes Ophthalmoskop anzugeben, bei welchem die Nachteile des Standes der Technik zumindest teilweise behoben sind, und welches sich insbesondere durch eine reduzierte Aberration auszeichnet und reproduzierbare sowie vergleichbare Abbildungen des Auges ermöglicht.

Diese Aufgabe wird durch die Merkmale des Anspruches 1 gelöst.

Es ist demnach erfindungsgemäß vorgesehen, dass das wenigstens eine Objektiv eine konvex gekrümmte Schärfefläche aufweist, wobei eine konvex gekrümmte Bildfläche eines Auges durch das wenigstens eine Objektiv scharf auf die wenigstens eine Vorrichtung zur Umwandlung abbildbar ist, wobei der gesamte sichtbare Bereich des Auges scharf auf die wenigstens eine Vorrichtung zur Umwandlung abbildbar ist und wobei das Ophthalmoskop eine gepulste Beleuchtung umfasst.

Der Fachterminus Schärfefläche eines Objektivs bezeichnet jene Fläche, welche bei einer Projektion einer Objektebene durch das Objektiv generiert wird. Die Schärfefläche kann herangezogen werden, um eine Bildfläche einer Objektfläche auf einer Vorrichtung zur Umwandlung geändert darzustellen. Ist die Objektfläche konvex gekrümmt, so ist ohne eine Linsenanordnung die Bildfläche ebenfalls konvex gekrümmt.

Durch eine konvex gekrümmte Schärfefläche der Linsenanordnung wird die konvex gekrümmte Bildfläche verändert. Eine stärker konvex gekrümmte Bildfläche kann somit in eine weniger konvex gekrümmte Bildfläche transformiert werden.

Der Fachterminus Objektfläche beschreibt die Oberflächengeometrie eines Objektes, wobei entgegen einer flachen Objektebene in dieser Verallgemeinerung eine Krümmung in der Oberflächengeometrie des Objektes vorhanden sein kann. Der Fachterminus Bildfläche beschreibt die Oberflächengeometrie der Abbildung des Objektes, wobei entgegen einer flachen Bildebene in dieser Verallgemeinerung eine Krümmung in der Oberflächengeometrie der Abbildung des Objektes vorhanden sein kann. Diese Betrachtung ist nicht auf Objektive mit optischen Elementen beschränkt.

Der Begriff scharf ist in diesem Kontext der Ophthalmoskopie dahingehend definiert, dass die Abbildung eines Objektes eine hohe Auflösung aufweist, wobei ein Kontrast eines Linienpaares zwischen getrennten, benachbarten Pixeln auf der wenigstens einen Vorrichtung zur Umwandlung von zumindest 20 %, vorteilhafter zumindest 30 %, besteht. In der geometrischen Optik können nur diejenigen Punkte des Objektes als scharfe Bildpunkte in der Vorrichtung zur Umwandlung wiedergegeben werden, welche auf einer Bildebene liegen, die sich in einer Gegenstandsweite respektive Objektweite zur Linse befinden. Handelt es sich bei der Bildebene um eine konvex gekrümmten Bildfläche, da auch die Objektfläche des Objektes konvex gekrümmt ist, so muss eine Anordnung von Linsen die Krümmung der Bildfläche kompensieren, um eine scharfe Abbildung des Objektes auf der Vorrichtung zur Umwandlung generieren zu können.

Durch die konvex gekrümmte Schärfefläche des wenigstens einen Objektivs wird ermöglicht, komplexe konvexe Geometrien eines Auges im sichtbaren Wellenlängenbereich von Licht scharf auf die wenigstens eine Vorrichtung zur Umwandlung zu projizieren, wobei die scharfe Abbildung auf der wenigstens einen Vorrichtung zur Umwandlung durch die konvex gekrümmte Schärfefläche an die gegebene Oberflächengeometrie der wenigstens einen Vorrichtung zur Umwandlung angepasst werden kann.

Hinzu kommt die positive Eigenschaft, dass die konvex gekrümmte Schärfefläche des wenigstens einen Objektivs individuell auf die Gegebenheiten einer spezifischen Augengeometrie adaptiertbar ist, wobei die gesamte für das wenigstens eine Objektiv sichtbare Oberfläche des Auges scharf auf die wenigstens eine Vorrichtung zur Umwandlung abgebildet werden kann.

Da der gesamte sichtbare Bereich des Auges scharf auf die wenigstens eine Vorrichtung zur Umwandlung abbildbar ist, wird zudem eine Standardisierung von Abbildungen des Auges sowie ein Vergleich einer Vielzahl von Abbildungen des Auges ermöglicht und eine Kategorisierung anhand augenspezifischer Charakteristika kann erfolgen. Eine Analyse der Abbildung des Auges wird dadurch erheblich begünstigt. Auch bidirektionale Messungen an der Abbildung des Auges werden ermöglicht, welche durch eine Abbildung einer Ringzone des Auges nicht durchführbar wären.

Umfasst das Ophthalmoskop mit wenigstens einer Linse wenigstens einen Spiegel, handelt es sich um ein sogenanntes katadioptrisches System, wobei durch den wenigstens einen Spiegel die scharfe Abbildung des Auges besonders begünstigt wird.

Bei dem wenigstens einen Spiegel kann es sich beispielsweise um einen Freiformspiegel handeln.

Es sind auch Ophthalmoskope möglich, deren wenigstens ein Objektiv nur durch Spiegel gebildet wird und ohne Linsen auskommt.

Durch den wenigstens einen Spiegel können Strahlengänge von dem Auge auf die wenigstens eine Vorrichtung zur Umwandlung besonders begünstigt manipuliert werden, um eine scharfe Abbildung des Auges zu verbessern.

Im Folgenden beziehen sich die Erläuterungen auf die Abbildung eines Auges. Im Allgemeinen sind jedoch auch beide Augen durch das Ophthalmoskop synchron respektive gleichzeitig in Stereo abbildbar.

Wie eingangs ausgeführt, wird Schutz auch begehrt für das Ophthalmoskop mit einer Spaltlampe. Es wird Schutz auch begehrt für die Verwendungen des Ophthalmoskops am menschlichen Auge.

Weiters wird Schutz auch begehrt für ein Verfahren zur Untersuchung von Augen, umfassend die Schritte
- Grobjustage eines Patienten in einer Haltevorrichtung, insbesondere mit einer Genauigkeit unterhalb 3 mm,
- Erkennung einer Pupille und/oder eines Scheitels einer Kornea des Auges über ein Bildverarbeitungsprogramm, wobei insbesondere vorgesehen ist, dass das Bildverarbeitungsprogramm einen Autofukusalgorithmus aufweist,
- Aufnahme von wenigstens einer Abbildung des Auges durch ein solches Ophthalmoskop, wobei die wenigstens eine Abbildung des Auges auf wenigstens einer Vorrichtung zur Umwandlung aufgenommen wird.

Der Autofokusalgorithmus kann eine Kontrastoptimierung und/oder eine Lagemessung vornehmen, wobei die Lagemessung beispielsweise durch eine seitliche Kamera zur Aufnahme eines Scheitels des Auges als Linie oder eine Entfernungsmessung z.B. über einen Laser durchgeführt werden kann.

Des Weiteren wird Schutz auch begehrt für ein solches Ophthalmoskop mit einem Computerprogrammprodukt, umfassend Befehle, die bei der Ausführung durch eine Recheneinheit diese veranlassen, für das Ophthalmoskop zur Untersuchung von Augen
aus einer Speichereinheit, welche mit der Recheneinheit in einer Datenverbindung steht oder in eine solche bringbar ist, wenigstens eine Abbildung eines Auges zu klassifizieren, wobei eine Klassifikation basierend auf einer Blutgefäßstruktur und/oder einer Hornhautstruktur und/oder einer Narbenstruktur und/oder einer Augenhöhlenstruktur generiert wird.

Eine Blutgefäßstruktur kann beispielsweise ein Indikator für eine Rötung des Auges und/oder Trockenheit des Auges herangezogen werden.

Vorteilhafte Ausführungsformen der Erfindung sind in den abhängigen Ansprüchen definiert.

Gemäß einer vorteilhaften Ausgestaltung der Erfindung ist vorgesehen, dass der Chip als CMOS-Sensor ausgebildet ist.

Bei dem Chip kann es sich beispielsweise um einen CCD-Sensor handeln. Besonders bevorzugt liegt der Chip in Form eines CMOS-Sensors vor. Der CMOS-Sensor umfasst komplementäre Metall-Oxid-Halbleiterbauelemente (complementary metal-oxide-semiconductors) und kann als aktiver Pixelsensor oder als passiver Pixelsensor vorliegen.

Durch einen Chip und insbesondere durch einen CMOS-Sensor kann eine Abbildung des Auges komfortabel innerhalb kürzester Zeit ausgelesen und zu einer Speicherung zur Verfügung gestellt werden, wodurch pro Zeitintervall die mögliche Anzahl an durch die wenigstens eine Vorrichtung zur Umwandlung aufzunehmenden Abbildungen des Auges erhöht wird.

In Kombination mit einer gepulsten Beleuchtung und/oder einer hohen Transmission des wenigstens einen Objektivs kann die Zeit pro Abbildung des Auges durch das Ophthalmoskop zusätzlich reduziert werden. Zudem kann eine hohe Helligkeit ohne einer Blendung des Patienten erwirkt werden. Des Weiteren wird ein Bildrauschen reduziert.

Vorteilhafter Weise ist vorgesehen, dass die wenigstens eine Vorrichtung zur Umwandlung wenigstens einen Bayerfilter umfasst.

Der Fachterminus Bayerfilter beschreibt einen Farbfilter, wobei die drei Grundfarben Rot, Grün und Blau und gegebenenfalls die Farbe Weiß in Form von Pixeln in einer Schachbrettstruktur angeordnet sind.

Durch wenigstens einen Bayerfilter wird ermöglicht, dass die wenigstens eine Vorrichtung zur Umwandlung besonders begünstigt Farbinformationen der Abbildung des Auges aufnehmen kann.

Der wenigstens eine Bayerfilter kann eine Aufteilung in rote, grüne und blaue Pixel generieren. Bevorzugt wird breitbandiges Licht mit einer Halbwertsbreite im Bereich von 70 nm bis 130 nm durch den Bayerfilter durchgelassen. Maxima einer Quanteneffizienz des Bayerfilters liegen im Bereich zwischen 450-455 nm für blaue, 535-545 nm für grüne und 610-615 nm für rote Pixel.

Als günstig hat sich erwiesen, dass wenigstens zwei Linsen in Form wenigstens einer Luftlinse ausgebildet sind, wobei eine Petzvalsumme der wenigstens einen Luftlinse negativ ist.

Der Begriff Luftlinse beschreibt eine Linsenanordnung, welche eine negative Petzvalsumme aufweist. Die Petzvalsumme entspricht einem reziproken Radius einer Petzvalfläche, wobei der reziproke Radius der Petzvalfläche durch die Summe aller Kehrwerte der beteiligten Brechungsindizes der (dünnen) Linsen multipliziert mit der jeweiligen Brennweite der Linse in Strahlrichtung definiert ist.

Durch wenigstens eine Luftlinse wird eine Abbildung einer konvex gekrümmten Oberfläche auf eine planare Oberfläche mit einem Objektiv ermöglicht, welches eine geringere Anzahl an Linsen umfasst.

Des Weiteren wird die Abbildung des Auges mit einer komplexen Geometrie, welche beispielsweise durch variable Krümmungsradien bedingt sein kann, besonders begünstigt.

Der exakte numerische Wert der benötigten Petzvalsumme für eine Abbildung einer konvex gekrümmten Oberfläche auf eine planare Oberfläche kann beispielsweise über die weiteren Linsen im Objektiv oder Distanzen zwischen der wenigstens einen Vorrichtung zur Umwandlung und dem abzubildenden Objekt errechnet werden, wobei die Brechungsindizes und die Brennweiten des Objektivs an die Anforderungen des Ophthalmoskops dahingehend angepasst werden können.

Besonders bevorzugt ist vorgesehen, dass innerhalb der wenigstens einen Luftlinse ein Gasgemisch mit einem Brechungsindex kleiner 1.3, bevorzugt kleiner 1.1 angeordnet ist, wobei vorzugsweise vorgesehen ist, dass das Gasgemisch zumindest ein Edelgas, besonders bevorzugt Argon, Stickstoff und/oder Luft enthält, besonders bevorzugt in Form des Edelgases, von Stickstoff oder von Luft vorliegt.

Im Allgemeinen sind auch Linsenanordnungen möglich, welche über gel-, flüssig- und/oder gummibasierte Einschlüsse eine Petzvalsumme mit dem erforderlichen Wert aufweisen. Besonders bevorzugt wird jedoch ein Gasgemisch vollständig durch die wenigstens zwei Linsen umschlossen, wobei sich Luft aufgrund des niedrigen Brechungsindex und der hohen Verfügbarkeit als besonders effektiv und kostenschonend erwiesen hat, wobei ein besonders kompaktes Ophtalmoskop gebildet werden kann. Anderweitige Gasgemische in der wenigstens einen Luftlinse sind jedoch ebenfalls denkbar.

Gemäß einer vorteilhaften Ausführungsform der Erfindung ist vorgesehen, dass zwischen einer sensorseitigen Linse und einer objektseitigen Linse ein linsenförmiger Fluideinschluss angeordnet ist, wobei die Petzvalsumme der sensorseitigen Linse, der objektseitigen Linse und des Fluideinschlusses negativ ist.

Da der Brechungsindex des wenigstens einen Fluideinschlusses einfach und kostenschonend durch die Zusammensetzung eines Fluides adjustiert werden kann, wird eine hohe Flexibilität hinsichtlich der Anforderungen an das wenigstens eine Objektiv ermöglicht.

Weiters ist der Brechungsindex eine Fluides wie beispielsweise Luft in der Regel viel niedriger als jener Brechungsindex von Materialien für Linsen wie beispielsweise optisches Glas, wodurch die Petzvalsumme einer Linsenanordnung besonders effizient und/oder mit einem geringen Materialaufwand auf einen negativen Wert eingestellt werden kann.

Als vorteilhaft hat sich erwiesen, dass die wenigstens eine Luftlinse derart ausgebildet ist, dass objektseitig eine Linse mit einem ersten Brechungsindex und sensorseitig eine Linse mit einem zweiten Brechungsindex angeordnet ist, wobei der zweite Brechungsindex höher als der erste Brechungsindex ist und/oder die wenigstens eine Luftlinse bikonvex ausgebildet ist.

Bei einer bikonvexen Luftlinse ist eine Kombination der Brennweiten besonders günstig für eine negative Petzvalsumme.

Ist der erste Brechungsindex niedriger als der zweite Brechungsindex, kann sich dies in Bezug auf negative Terme in der Petzvalsumme begünstigend auf das Ophthalmoskop auswirken.

Eine vorteilhafte Variante besteht darin, dass genau zwei voneinander beabstandete Luftlinsen vorhanden sind.

Durch zwei beabstandete Luftlinsen wird ermöglicht, dass zwischen den zwei Luftlinsen zusätzliche optische Elemente wie beispielsweise Linsen im Objektiv angeordnet werden können.

Ein Strahlgang vom Auge zur Vorrichtung zur Umwandlung kann somit je nach Anforderung an die Abbildung des Auges auf der Vorrichtung zur Umwandlung durch eine Anordnung und/oder Ausgestaltung der Luftlinsen und/oder durch zusätzliche optische Elemente manipuliert werden.

Besonders bevorzugt ist, dass das wenigstens eine Objektiv und/oder eine objektseitige Abbildung und/oder ein objektseitiger Strahlengang im Wesentlichen telezentrisch, vorzugsweise perizentrisch, ausgebildet ist.

Bei herkömmlichen entozentrischen Objektiven wird ein Einfallswinkel in einem Randbereich einer Sklera sehr flach, wodurch eine tangentiale Auflösung in hohem Maße abnimmt.

Eine günstigere tangentiale Auflösung wird durch ein telezentrisches Objektiv, eine telezentrische objektseitige Abbildung und/oder einen telezentrischen objektseitigen Strahlengang ermöglicht. Eine tangentiale Auflösung noch höheren Maßes wird durch ein perizentrisches Objektiv, eine perizentrische objektseitige Abbildung und/oder einen perizentrischen objektseitigen Strahlgang ermöglicht.

Bei einem Ausführungsbeispiel der Erfindung ist vorgesehen, dass eine Abbildung auf der wenigstens einen Vorrichtung zur Umwandlung farbig dargestellt ist.

Durch eine farbige Darstellung der Abbildung können feine Strukturen des Auges durch einen Benutzer des Ophthalmoskops besser erkannt werden.

Bevorzugt umfasst die wenigstens eine Vorrichtung zur Umwandlung einen Infrarot-Cut-Filter, wobei der Infrarot-Cut-Filter besonders bevorzugt Wellenlängen oberhalb von 620 nm abschneidet.

Gemäß einem bevorzugten Ausführungsbeispiel der Erfindung ist vorgesehen, dass eine sensorseitige numerische Apertur des wenigstens einen Objektivs im Bereich von 0,04 bis 0,1, vorzugsweise im Bereich von 0,06 bis 0,08, liegt.

Die numerische Apertur gibt ein Vermögen eines optischen Elementes an, Licht zu fokussieren. Die numerische Apertur ist das Produkt des Sinus eines halben objektseitigen Öffnungswinkels und dem Brechungsindex eines Materials zwischen Objektiv und einem Fokus.

Durch die sensorseitige numerische Apertur im Bereich von 0,04 bis 0,1 wird eine Auflösung des Ophthalmoskops ermöglicht, welche nicht durch Beugungserscheinungen beeinträchtigt ist.

Als günstig hat sich erwiesen, dass das wenigstens eine Objektiv eine Blende aufweist, wobei die Blende einen Radius im Bereich von 2,5 mm bis 3,5 mm, vorzugsweise im Bereich von 2,9 bis 3,3, aufweist.

Eine besonders bevorzugte Vorrichtung zur Umwandlung umfasst eine Auflösung im Bereich von 4,88 µm. Diese wird durch eine Blendenradius zwischen 2,5 mm und 3,5 mm ermöglicht.

Weiters ist bevorzugt vorgesehen, dass das Ophthalmoskop genau acht Linsen und/oder genau zwei Planscheiben umfasst.

Um eine komplexe Geometrie wie die Oberfläche des Auges auf eine planare Oberfläche zu projizieren, hat sich in Bezug auf die Schärfe der Abbildung des Auges und unter der Rücksicht der baulichen Größen sowie Kosten und Gewicht ein Ophthalmoskop mit acht Linsen und zwei Planscheiben als besonders bevorzugt erwiesen.

Die Oberfläche des Auges weist konvexe Teilbereiche unterschiedlicher Krümmungen auf. Die Oberfläche des Auges kann als Freiformfläche angesehen werden, welche im Wesentlichen rotationssymmetrisch ausgebildet sein kann.

Alternativ ist möglich, dass wenigstens eine Linse asphärisch ausgebildet ist.

Asphärische Linsen ermöglichen es, Aberration (Abbildungsfehler) durch die geometrische Struktur der asphärischen Linse besonders effektiv zu reduzieren.

Gemäß einer vorteilhaften Ausgestaltung der Erfindung ist vorgesehen, dass wenigstens ein Leuchtpunktabsehen in dem wenigstens einen Objektiv, vorzugsweise zentral auf einer optischen Achse des wenigstens einen Objektivs verortet, angeordnet ist.

Durch das wenigstens eine Leuchtpunktabsehen kann eine Fixation des Auges des Patienten erwirkt werden.

Bevorzugt ist das wenigstens eine Leuchtpunktabsehen im Wesentlichen auf einer Brennfläche wenigstens einer Linse, bevorzugt im Brennpunkt als Schnittpunkt der Brennfläche mit der optischen Achse, angeordnet.

Dadurch wird das Auge entspannt und kann auf einen im Unendlichen verorteten Punkt akkommodieren.

Durch das wenigstens eine Leuchtpunktabsehen wird ein Referenzpunkt für das Auge geboten, welcher während einer Benutzung des Ophthalmoskops vom Auge anvisiert werden kann. Bei mehreren Abbildungen des Auges durch das Ophthalmoskop wird dadurch eine Referenz geboten, um über eine Zeitspanne der Aufnahme der Abbildungen die gleiche Orientierung des Auges abbilden zu können. Bei mehreren Abbildungen von unterschiedlichen Augen wird zudem ermöglicht, diese miteinander zu vergleichen, da das wenigstens eine Leuchtpunktabsehen eine im Wesentlichen standardisierte Ausrichtung des Auges gewährleistet.

Gemäß einer vorteilhaften Ausführungsform der Erfindung ist vorgesehen, dass das wenigstens eine Leuchtpunktabsehen wenigstens eine diffraktive Struktur umfasst, wobei die wenigstens eine diffraktive Struktur eine Ausdehnung im Bereich von 25 µm bis 75 pm, vorzugsweise im Bereich von 40 µm bis 55 µm, aufweist.

Besonders bevorzugt wird die wenigstens eine diffraktive Struktur des wenigstens einen Leuchtpunktabsehens seitlich mit wenigstens einer LED beliebiger Farbe beleuchtet, wobei das Licht der wenigstens einen LED über die wenigstens eine diffraktive Struktur auf das abzubildende Auge trifft.

Gemäß einer vorteilhaften Ausgestaltung der Erfindung ist vorgesehen, dass das wenigstens eine Leuchtpunktabsehen sensorseitig durch eine Maske abgedeckt ist.

Durch die Maske wird ein störendes Falschlicht, welches nicht für die Abbildung des Auges durch die wenigstens eine Vorrichtung zur Umwandlung herangezogen wird, reduziert.

Gemäß einer vorteilhaften Ausgestaltung der Erfindung ist vorgesehen, dass das Gehäuse wenigstens einen Randzylinder umfasst, wobei der wenigstens eine Randzylinder, vorzugsweise im Bereich des wenigstens einen Leuchtpunktabsehens, geschwärzt ausgebildet ist.

Durch den wenigstens einen geschwärzten Randzylinder wird für die Abbildung des Auges störendes Streulicht reduziert.

Vorteilhafter Weise ist vorgesehen, dass das Licht des Auges in chronologischer Reihenfolge durch eine Objektivlinse, das Leuchtpunktabsehen, eine Meniskuslinse, eine objektseitige Luftlinse, eine objektseitige Zerstreuungslinse, die Blende, eine Sammellinse, einen Achromat, eine sensorseitige Meniskuslinse, eine sensorseitige Zerstreuungslinse und ein Schutzglas transmittierbar ist, wobei das Licht des Auges anschließend auf die wenigstens eine Vorrichtung zur Umwandlung treffbar ist.

Dadurch wird ein Strahlengang von dem Auge zur wenigstens einen Vorrichtung zur Umwandlung insbesondere bei komplexen Oberflächengeometrien des Auges und/oder diffizilen Strukturen, welche auf der Oberfläche des konvex gekrümmten Objektes gegebenenfalls vorhanden sind, besonders effektiv derart durch das Objektiv abgelenkt, dass auf der wenigstens einen Vorrichtung zur Umwandlung ein scharfes Abbild des Auges erfolgt.

Als günstig hat sich erwiesen, dass die Objektivlinse und/oder die objektseitige Meniskuslinse und/oder der Achromat Flintglas umfassen.

Flintglas zeichnet sich durch eine Abbezahl kleiner als 50 aus und weist einen höheren Brechungsindex auf als Kronglas. Flintglas weist zudem eine gleichmäßige optische Dispersion auf.

Gemäß einer vorteilhaften Ausführungsform der Erfindung ist vorgesehen, dass die Sammellinse und/oder der Achromat Kronglas umfassen.

Kronglas zeichnet sich bei farbkorrigierten, optischen Gläsern durch eine Abbezahl größer als 50, wodurch eine Dispersion gering gehalten wird, und einem Brechungsindex unterhalb von Flintglas aus.

Als vorteilhaft hat sich erwiesen, dass wenigstens eine Haltevorrichtung für einen Kopf, vorzugsweise mit einer Kinnauflage und/oder einer Stirnstütze, vorgesehen ist.

Durch eine Haltevorrichtung wird eine Positionsungenauigkeit des Auges während wenigstens einer Abbildung durch das Ophthalmoskop reduziert und/oder die wenigstens eine Abbildung des Auges vereinheitlicht. Eine Kinnauflage und/oder eine Stirnstütze kann die Positionsgenauigkeit des Auges weiter erhöhen, um die Abbildungen des Auges auch für eine Normierung von Abbildungen heranziehen zu können.

Eine vorteilhafte Variante besteht darin, dass die Haltevorrichtung von der wenigstens einen Vorrichtung zur Umwandlung im Bereich von 30 mm bis 200 mm, vorzugsweise im Bereich von 40 mm bis 100 mm, beabstandet ist.

Wenn die wenigstens eine Vorrichtung zur Umwandlung in Datenverbindung mit wenigstens einem Computer bringbar ist, wobei der wenigstens eine Computer in Verbindung mit einer Datenbank bringbar ist, können die durch die Vorrichtung zur Umwandlung aufgenommene Abbildungen digital abgespeichert werden, um anschließend für Vergleiche oder Analysen herangezogen werden können.

Besonders bevorzugt ist, dass am Gehäuse wenigstens eine Beleuchtung angeordnet ist, wobei die Illumination des Auges über die wenigstens eine Beleuchtung bereichsweise durch das wenigstens eine Objektiv verdeckt ist.

Durch die wenigstens eine Beleuchtung an sich wird eine ausreichende Belichtung des Auges ermöglicht, wobei durch die teilweise Abschirmung der wenigstens einen Beleuchtung durch das wenigstens eine Objektiv verhindert wird, dass direkt an einer glatten Oberfläche des Auges reflektiertes Licht nicht durch das wenigstens eine Objektiv auf die wenigstens eine Vorrichtung zur Umwandlung gelangt.

Dadurch wird eine im Wesentlichen homogene und/oder isotrope Belichtung des Auges ermöglicht, wobei partielle Bereiche einer Überbelichtung ohne zusätzliche Komponenten des Ophthalmoskops verhindert werden.

Besonders bevorzugt ist die wenigstens eine Beleuchtung in Form einer LED-Beleuchtung ausgebildet.

Die wenigstens eine Beleuchtung wird gepulst betrieben, um einen Benutzer des Ophthalmoskops und/oder einen Patienten nicht zu blenden. Die Pulsdauer der wenigstens einen Beleuchtung kann mit der wenigstens einen Vorrichtung zur Umwandlung synchronisiert werden, wobei die wenigstens eine Beleuchtung in einem Zeitintervall einer Belichtungszeit der wenigstens einen Vorrichtung zur Umwandlung leuchtet.

Die Belichtungszeit der wenigstens einen Vorrichtung zur Umwandlung kann in einem Bereich von 1 µs bis 5 s eingestellt werden. Bevorzugt ist eine Belichtungszeit respektive eine Pulsdauer zwischen 10 µs und 30 ms, besonders bevorzugt zwischen 20 µs und 200 µs. Dadurch wird eine Helligkeit der wenigstens einen Abbildung des Auges besonders begünstigt.

Die wenigstens eine Beleuchtung wird mit einem Pulsweitenmodulationssignal angesteuert, wobei bevorzugt eine Einschaltzeit der wenigstens einen Beleuchtung unterhalb von 10 % liegt. Dadurch kann bei einem gleichen subjektiven Helligkeitsempfinden des Patienten eine Beleuchtungsintensität um ein Zehnfaches erhöht werden.

Besonders bevorzugt ist wenigstens eine Beleuchtung, welche schwenkbar am Gehäuse gelagert ist, um eine Feinjustierung der Illumination des Auges zu gewährleisten.

Gemäß einer vorteilhaften Ausgestaltung der Erfindung ist vorgesehen, dass die wenigstens eine Beleuchtung in Form einer Weißlichtbeleuchtung, vorzugsweise mit einem sonnenlichtähnlichen Weißton mit einer charakteristischen Farbtemperatur zwischen 5000 K und 6000 K und/oder einem Farbwiedergabeindex von zumindest 95 %, und/oder einer fluoreszenten Beleuchtung, ausgestaltet ist.

Bei einer fluoreszenten Beleuchtung können Augen oder Tränenflüssigkeiten auf dem Auge, welche beispielsweise mit einem Fluoreszeinfarbstoff angereichert sind, besonders begünstigt abgebildet werden, da das Licht der fluoreszenten Beleuchtung von den Fluoreszeinfarbstoffen in einem bekannten Wellenlängenbereich emittiert werden und durch die wenigstens eine Vorrichtung zur Umwandlung aufgenommen werden kann.

Besonders bevorzugt ist, dass die fluoreszente Beleuchtung einen Wellenlängenbereich zwischen 450 nm und 510 nm und/oder zwischen 750 nm und 780 nm aufweist.

Dadurch wird eine Verwendung des Ophthalmoskops am menschlichen Auge unter der Verwendung der Farbstoffe Fluoreszein und/oder Indizyaningrün für eine Abbildung des Auges besonders begünstigt.

Somit sind Abbildungen des Auges möglich, welche spezifische Bereiche des Auges besonders hervorheben. Die fluoreszente Beleuchtung wird vom Auge beispielsweise bei einer Wellenlänge von 765 nm absorbiert, wobei eine anschließende Lichtemission beispielsweise durch Indozyaningrün bei 830 nm erfolgt.

Das Anreichern des Auges mit Fluoreszenzfarbstoff kann beispielsweise über ein Eintropfen von Fluoreszenzfarbstoff erfolgen. Eine Injektion des Fluoreszenzfarbstoffes beispielsweise in das Auge oder Blutgefäße des Körper des Patienten ist ebenfalls denkbar.

Bei einer Weißlichtbeleuchtung wird sichtbares Licht verwendet und ein mit Fluoreszenzfarbstoff angereichertes Auge ist für eine Abbildung auf der wenigstens einen Vorrichtung zur Umwandlung nicht notwendig. Die Verwendung einer Weißlichtbeleuchtung ist zudem kostengünstiger in der Anwendung.

Besonders bevorzugt ist das Ophthalmoskop derart ausgebildet, dass das Ophthalmoskop zumindest zwei Modi aufweist, wobei ein Modus die Aufnahme von Abbildungen des Auges über die Weißlichtbeleuchtung und ein weiterer Modus die Aufnahme von Abbildungen des Auges über die fluoreszente Beleuchtung ermöglicht. Das Ophthalmoskop kann in dieser Ausführungsform zwischen den Modi wechseln.

Als günstig hat sich erwiesen, dass wenigstens ein Fluoreszenzfilter zwischen der wenigstens einen Vorrichtung zur Umwandlung und dem Auge angeordnet ist.

Durch den wenigstens einen Fluoreszenzfilter, welcher besonders bevorzugt innerhalb des wenigstens einen Objektives in einem Fokus wenigstens einer Linse angeordnet ist, können spezifische Wellenlängenbereiche, welche für die Abbildung des Auges nicht notwendig sind, herausgefiltert werden.

Bei einem Ausführungsbeispiel der Erfindung ist vorgesehen, dass das Ophthalmoskop wenigstens einen Statusbildschirm umfasst, wobei auf dem wenigstens einen Statusbildschirm wenigstens eine elektronische Information visualisierbar ist.

Durch den wenigstens einen Statusbildschirm kann ein Patient in der Benutzung des Ophthalmoskops beispielsweise informiert werden, dass seine Augenlieder zu viel Oberfläche des Auges verdecken. Am Statusbildschirm kann auch beispielsweise eine Vorschau der Abbildung durch die wenigstens eine Vorrichtung zur Umwandlung angezeigt werden. Eine Anzeige von elektronischen Nachrichten auf dem wenigstens einen Statusbildschirm ermöglicht es dem Patienten beispielsweise, sein Auge derart zu positionieren, dass eine Abbildung des Auges möglichst scharf wird. Integrierte farbkodierte LEDs sind ebenfalls zur Informationsweitergabe an den Patienten und/oder Bediener des Ophthalmoskops denkbar.

Als günstig hat sich erwiesen, dass die wenigstens eine Abbildung des Auges auf der wenigstens einen Vorrichtung zur Umwandlung in wenigstens einen Computer, vorzugsweise in eine Datenbank auf dem wenigstens einen Computer, eingelesen und/oder gespeichert wird.

Durch die Speicherung der wenigstens einen Abbildung am Computer wird eine Vervielfältigung der wenigstens einen Abbildung sowie ein Vergleich von mehreren Abbildungen, gegebenenfalls unterschiedlicher Augen, ermöglicht.

Im Allgemeinen ist auch eine digitale Nachbearbeitung mit einer Software möglich, um die wenigstens eine Abbildung des Auges in abgeänderter Art und Weise darzustellen.

Besonders bevorzugt ist vorgesehen, dass Bewegungen des Auges unterhalb von 3 mm durch eine automatisierte Nachführung nachgeführt werden und/oder eine Öffnung eines Augenlides über das Bildverarbeitungsprogramm erkannt wird, wobei vorzugsweise vorgesehen ist, dass bei einer Öffnung unter 12 mm, vorzugsweise unter 16 mm, eine Anzeige an wenigstens einem Statusbildschirm angezeigt wird.

Dadurch wird ermöglicht, dass Bewegungen des Patienten und/oder Überdeckungen durch ein Augenlid die scharfe Abbildung des Auges nicht beeinträchtigen.

In einer bevorzugten Variante der Erfindung ist vorgesehen, dass die wenigstens eine Abbildung des Auges hinsichtlich augenspezifischer Charakteristika ausgewertet und/oder analysiert wird.

Dadurch wird eine Kategorisierung von Abbildungen des Auges effizienter und/oder eine Klassifizierung anhand spezifischer augencharakteristischer Parameter effektiver ermöglicht. Im Allgemeinen kann dies durch eine computergestützte Datenverarbeitungssoftware unterstützt sein.

Augenspezifische Charakteristika können beispielsweise Abmessungen und/oder Geometrien des Auges, eine Blutgefäßstruktur, eine Hornhautstruktur, eine Narbenstruktur und/oder eine Augenhöhlenstruktur darstellen.

Eine vorteilhafte Variante besteht darin, dass die wenigstens eine Abbildung des Auges am wenigstens einen Computer zur Standardisierung und/oder Normierung kategorisiert wird.

Durch eine Kategorisierung werden eine Suche nach augenspezifischen Merkmalen und/oder ein Vergleich ähnlicher augenspezifischer Merkmale in der wenigstens einen Abbildung des Auges erleichtert.

Bei einem Ausführungsbeispiel der Erfindung ist vorgesehen, dass das Auge eine Kontaktlinse und eine mit Fluoreszenzfarbstoff angereicherte Tränenflüssigkeit aufweist, wobei die Tränenflüssigkeit mit einer fluoreszenten Beleuchtung angestrahlt wird, die Tränenflüssigkeit Licht in einem Wellenlängenbereich zwischen 515 nm und 530 nm und/oder zwischen 825 nm und 835 nm emittiert und eine Verteilung des emittierten Lichtes der Tränenflüssigkeit zwischen der Kontaktlinse und dem Auge auf der wenigstens einen Vorrichtung zur Umwandlung aufgenommen wird.

Durch die abgebildete Verteilung der Tränenflüssigkeit durch die Abbildung des Auges, wobei die abgebildete Verteilung durch eine Lichtemission über die Fluoreszenzfarbstoffe (beispielsweise Fluoreszein und/oder Indizyaningrün) erwirkt wird, wird ermöglicht, Passungenauigkeiten der Kontaktlinse am Auge zu identifizieren.

Bei einer Ausgestaltung der Erfindung ist vorgesehen, dass das Auge wenigstens einen Fluoreszenzfarbstoff umfasst und eine Blutgefäßstruktur und/oder eine Lymphgefäßstruktur und/oder ein Hornhautepithel des Auges, vorzugsweise über den wenigstens einen Fluoreszenzfilter, auf der wenigstens einen Vorrichtung zur Umwandlung abgebildet und/oder auf dem wenigstens einen Computer gespeichert wird.

Die Blutgefäßstruktur, die Lymphgefäßstruktur und/oder das Hornhautepithel sind über sichtbares Licht abbildbar. Besonders begünstigt werden diese Strukturen durch die Verwendung der fluoreszenten Beleuchtung abgebildet, da der Kontrast zu weiteren Strukturen des Auges erhöht wird.

Als vorteilhaft hat sich erwiesen, dass der Fluoreszenzfarbstoff Fluoreszein und/oder Indizyaningrün umfasst.

Dadurch sind Strukturen des Auges besonders begünstigend durch die wenigstens eine Vorrichtung zur Umwandlung abbildbar.

Besonders bevorzugt ist, dass nach einem Zeitraum von zumindest einem Tag das Verfahren erneut durchgeführt wird.

Dadurch wird ermöglicht, einen zeitlichen Verlauf eines Zustandes des Auges ersichtlich zu machen.

Insbesondere findet dieses Verfahren Anwendung in der Telemedizin. Beispielsweise kann eine Abbildung eines Auges durch das Ophthalmoskop an einem ersten Standort aufgenommen werden und an einen zweiten Standort, welcher beispielsweise eine zentrale Augenarztpraxis darstellen kann, übermittelt werden.

Zu bestimmten Zeitintervallen können somit weitere Abbildungen des Auges aufgenommen werden und an den zweiten Standort gesendet werden, wo bei einer Speicherung eine Vielzahl an Abbildungen des Auges vorliegen, welche einen zeitlichen Verlauf des Zustandes des Auges erkennbar machen.

Beispielsweise können über Abbildungen des Auges in einer Datenbank Gefäßneubildungen über einen Zeitraum ersichtlich werden, wobei eine Lokalisierung und/oder ein Ausmaß der Gefäßneubildung erkannt werden kann.

Ein expliziter Augenarzttermin bei einem Ophthalmologen ist hierbei nicht mehr erforderlich. Der Ophthalmologe kann auf Basis der ermittelten Daten eine Beurteilung erstellen, wobei das Ophthalmoskop eine Assistenzfunktion im Sinne der Datenaufbereitung zur verbesserten Diagnose durch den Ophthalmologen bereitstellt.

Als vorteilhaft hat sich erwiesen, dass eine Bildüberlagerung mit automatischer Blutgefäßerkennung und/oder automatischer Limbuserkennung zur Kontrolle der Klassifikation heranziehbar ist.

Dadurch wird eine Analyse der Abbildungen des Auges besonders begünstigt.

Als günstig hat sich erwiesen, dass eine halbautomatische Läsionserkennung und/oder bidirektionale Messungen vornehmbar sind.

Besonders bevorzugt ist, dass standardisierte Abbildungen wenigstens einer Iris in der Speichereinheit abgespeichert werden.

Bei einem Ausführungsbeispiel der Erfindung ist vorgesehen, dass abhängig von einer Farbe zweier Iris eine Farbe einer Kontaktlinse und/oder einer künstlichen Iris ausgewählt wird, wobei die Farbe der Kontaktlinse und/oder der künstlichen Iris an eine der beiden Iris angepasst ist.

Bei unterschiedlichen Irisfarben können durch eine gefärbte Kontaktlinse und/oder einer gefärbten künstlichen Iris die unterschiedlichen Farbtöne der Iris ausgeglichen werden.

Bei einer Ausgestaltung der Erfindung ist vorgesehen, dass die Blutgefäßstruktur, vorzugsweise über künstliche Intelligenz, automatisch erkannt wird.

Beispielsweise kann über maschinelles Lernen besonders effektiv eine Blutgefäßstruktur des Auges erkannt respektive identifiziert werden. Die Blutgefäßstruktur kann anschließend für eine Analyse und/oder eine Kategorisierung herangezogen werden.

Als vorteilhaft hat sich erwiesen, dass über die Bildüberlagerung eine Quantifizierung von Veränderungen der Blutgefäßstruktur und/oder der Hornhautstruktur berechnet wird.

Bei einer Vielzahl von Abbildungen des Auges kann über die Bildüberlagerung erkannt werden, inwiefern sich augenspezifische Charakteristika wie beispielsweise die Blutgefäßstruktur über einen zeitlichen Verlauf verändert haben.

Die Quantifizierung kann hierbei die zeitliche Veränderung in Form einer Kennzahl oder augenspezifischer Parameter wie Augenrötung angeben.

Besonders bevorzugt ist vorgesehen, dass eine Rotpixeldichtemessung vorgenommen wird, wobei die Rotpixelmessung einen gesamten Rotanteil der wenigstens einen Abbildung integriert und/oder einen Flächenanteil der Blutgefäßstruktur an der wenigstens einen Abbildung errechnet.

Wird der gesamte Rotanteil der Pixel der wenigstens einen Abbildung integriert, kann schnell und simpel ein Maß für eine Augenrötung angegeben werden.

Wird der Flächenanteil der Blutgefäßstruktur errechnet, so kann das Maß der Augenrötung exakter angegeben werden. Die Blutgefäßstruktur kann beispielsweise über einen Schwellwert des Rotanteils der Pixel in der Abbildung des Auges ermittelt werden.

Wenn das Computerprogrammprodukt eine automatische Läsionserkennung des Auges umfasst, können Anomalien des Auges automatisch erkannt werden und beispielsweise über eine Visualisierung angezeigt werden.

Eine vorteilhafte Variante besteht darin, dass eine Öffnung des Auges von zumindest 12 mm, vorzugsweise zumindest 16 mm, erkannt wird und bei einer Öffnung unter 12 mm, vorzugsweise unter 16 mm, eine elektronische Information an den wenigstens einen Statusbildschirm übermittelt wird.

Dadurch wird ermöglicht, dass das Auge bei einer Abbildung des Auges nicht in hohem Maße durch das Augenlid verdeckt wird. Über die elektronische Information wird dem Patienten in der Benutzung des Ophthalmoskops mitgeteilt, das Auge weiter zu öffnen, um mehr Aspekte der Augenoberfläche abbilden zu können.

Kleinere Fehlstellungen des Auges können durch das Ophthalmoskop beispielsweise durch eine Nachführung korrigiert werden, wobei bei zu hohen Fehlstellungen - beliebiger Art - der Statusbildschirm eine nicht hinreichende Ausrichtung und/oder Freilage des Auges anzeigt.

Ist eine scharfe Abbildung des Auges möglich, kann über den wenigstens einen Statusbildschirm dem Patienten, vorzugsweise über eine LED und/oder eine Visualisierung und/oder eine elektronische Nachricht, mitgeteilt werden, dass die scharfe Abbildung des Auges bei Beibehaltung der Position durchgeführt werden kann. Eine Vorschau der Abbildung des Auges über den wenigstens einen Statusbildschirm ist ebenfalls möglich.

Das Ophthalmoskop kann automatisch eine Abbildung des Auges durchführen, wenn das Auge richtig positioniert ist und nicht großflächig verdeckt ist. Zudem kann eine Blickrichtung abseits des wenigstens einen Leuchtpunktabsehens erkannt und/oder an dem wenigstens einen Statusbildschirm angezeigt werden.

Weitere Einzelheiten und Vorteile der vorliegenden Erfindung werden anhand der Figurenbeschreibung unter Bezugnahme auf die in den Zeichnungen dargestellten Ausführungsbeispiele im Folgenden näher erläutert. Darin zeigen:
- Fig. 1a-1c: ein Ophthalmoskop gemäß einem bevorzugten Ausführungsbeispiel mit einer Haltevorrichtung,
- Fig. 2a-2c: das Ophthalmoskop gemäß dem in Fig. 1a-1c gezeigten Ausführungsbeispiel mit einer Spaltlampe,
- Fig. 3a-3d: Strahlengänge von Licht zwischen einem Auge und einer Vorrichtung zur Umwandlung, wobei die Strahlengänge ein Objektiv durchlaufen,
- Fig. 4: ein Objektiv mit einer Beleuchtung in Richtung eines Auges in einer schematisch angedeuteten Ansicht von der Seite,
- Fig. 5: das Ophthalmoskop gemäß dem in Fig. 1a gezeigten Ausführungsbeispiel in Datenverbindung mit einem Computer und einem Flussdiagramm,
- Fig. 6a: eine nicht erfindungsgemäße Abbildung einer konkaven Geometrie durch eine Linse mit einem dargestellten Zusammenhang zwischen einer Schärfefläche, einer Bildfläche, einer objektseitiger Abbildung und einer Abbildung,
- Fig. 6b: ein Objektiv eines Opthalmoskops mit drei Spiegeln während einer Aufnahme einer Abbildung eines Auges auf eine Vorrichtung zur Umwandlung,
- Fig. 7a-7b: eine Blutgefäßstruktur in einer Abbildung eines menschlichen Auges und eine durch ein Computerprogrammprodukt identifizierte Blutgefäßstruktur über die Abbildung des Auges.

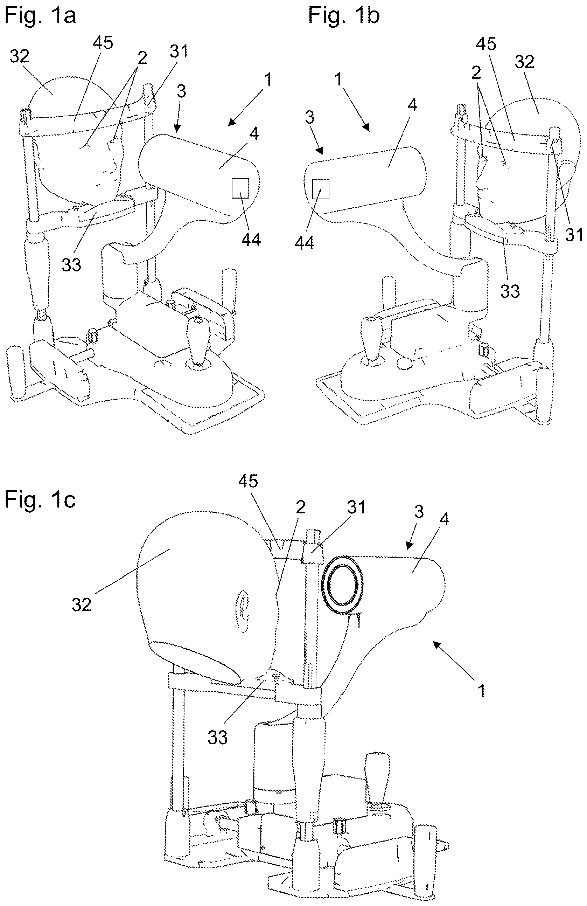

Fig. 1a zeigt ein Ophthalmoskop 1 zur Untersuchung von Augen 2 umfassend ein Gehäuse 3 und eine Vorrichtung zur Umwandlung 4 von Licht in ein elektrisches Signal (in der Darstellung nicht ersichtlich).

Es ist eine Haltevorrichtung 31 für einen Kopf 32 vorhanden, wobei an der Haltevorrichtung 31 eine Kinnauflage 33 und eine Stirnstütze 45 angeordnet ist. Durch die Haltevorrichtung 31 und die Kinnauflage 33 sowie die Stirnstütze 45 wird der Kopf 32 derart zum Ophthalmoskop 1 ausgerichtet, dass das Auge 2 für zumindest eine Abbildung 16 durch das Ophthalmoskop 1 ortsfest positioniert ist.

Die Haltevorrichtung 31 ist von der wenigstens einen Vorrichtung zur Umwandlung 4 zwischen 30 mm und 200 mm beabstandet, wobei die Beabstandung durch einen Bediener des Ophthalmoskops 1 adjustiert werden kann.

Das Ophthalmoskop 1 wird am menschlichen Auge 2 verwendet.

Fig. 1b zeigt das Ophthalmoskop 1 nach Fig. 1a um eine vertikale Achse um 90 Grad in eine Richtung geschwenkt. Fig. 1c zeigt das Ophthalmoskop 1 nach Fig. 1a um eine vertikale Achse um 90 Grad in die entgegengesetzte Richtung geschwenkt.

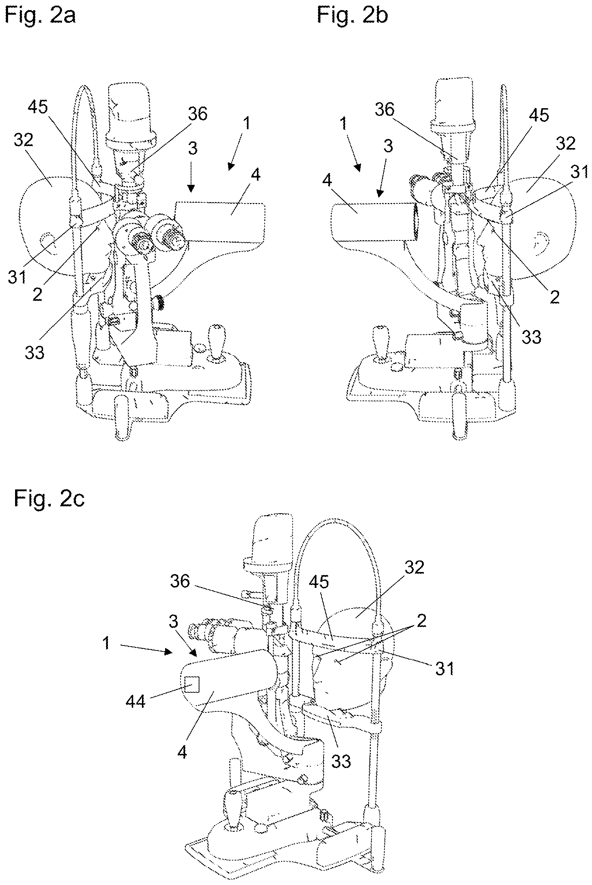

Fig. 2a zeigt das Ophthalmoskop 1 gemäß Fig. 1a mit einer Spaltlampe 36.

Eine Abbildung 16 auf der wenigstens einen Vorrichtung zur Umwandlung 4 kann farbig dargestellt werden. Eine sensorseitige numerische Apertur des Objektivs 5 liegt im Bereich von 0,04 bis 0,1. Das Objektiv 5 weist eine Blende auf, wobei die Blende während einem Abbilden des Auges 2 durch das Ophthalmoskop 1 einen Radius im Bereich von 2,5 mm bis 3,5 mm aufweist (aus Übersichtlichkeitsgründen nicht dargestellt).

Das Objektiv kann auch eine objektseitige numerische Apertur aufweisen, vorzugsweise im Bereich von 0,04 und 0,1.

Fig. 2b zeigt das Ophthalmoskop 1 nach Fig. 2a um eine vertikale Achse um 180 Grad geschwenkt. Fig. 2c zeigt das Ophthalmoskop 1 nach Fig. 2a in einer perspektivischen Ansicht von vorne.

Weist das Auge 2 eine Kontaktlinse und eine mit Fluoreszenzfarbstoff als Fluoreszenzfarbstoff angereicherte Tränenflüssigkeit auf, wobei die Tränenflüssigkeit mit einer fluoreszenten Beleuchtung 35 angestrahlt wird, kann die Tränenflüssigkeit Licht in einem Wellenlängenbereich zwischen 515 nm und 530 nm emittieren und eine Verteilung des emittierten Lichtes der Tränenflüssigkeit zwischen der Kontaktlinse und dem Auge 2 wird auf der wenigstens einen Vorrichtung zur Umwandlung 4 aufgenommen. Handelt es sich bei dem Fluoreszenzfarbstoff um Indozyaningrün, so weist das emittierte Licht eine Wellenlänge im Bereich von 825 nm bis 835 nm auf.

Wenn das Auge 2 wenigstens einen Fluoreszenzfarbstoff umfasst, kann eine Blutgefäßstruktur 46 und/oder eine Lymphgefäßstruktur und/oder ein Hornhautepithel des Auges 2 auf der wenigstens einen Vorrichtung zur Umwandlung 4 abgebildet werden. Besonders bevorzugt wird hierbei ein Fluoreszenzfilter 43 verwendet (aus Übersichtlichkeitsgründen nicht dargestellt). Die Abbildung 16 kann anschließend auf einem Computer 34 gespeichert werden.

Das Ophthalmoskop 1 umfasst einen Statusbildschirm 44, wobei auf dem Statusbildschirm 44 eine elektronische Information visualisiert ist.

Die Abbildungen 16 können nach einem gewünschten Intervall wiederholt werden.

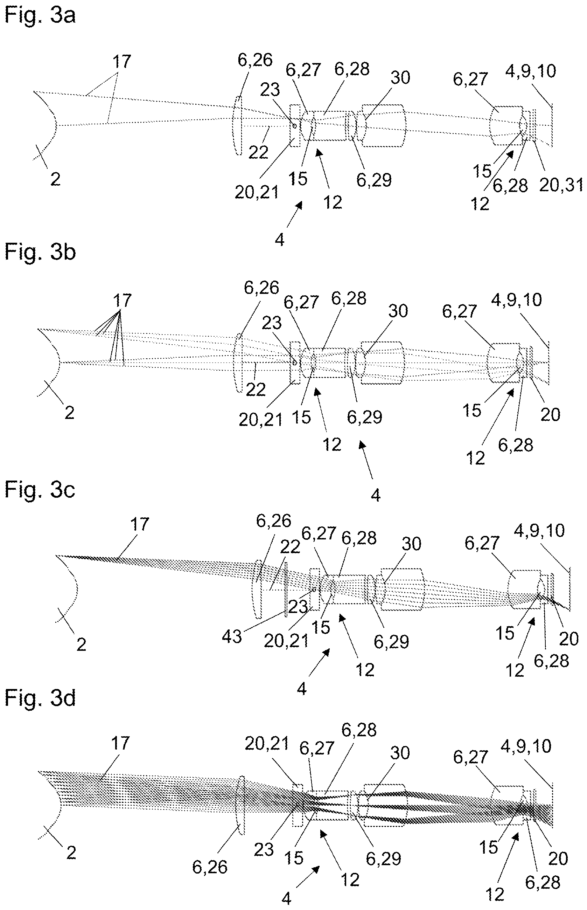

Fig. 3a zeigt eine Schnittdarstellung einer Oberflächengeometrie eines Auges 2, eines Objektivs 5 sowie einer Vorrichtung zur Umwandlung 4. Die Vorrichtung zur Umwandlung 4 ist in Form eines planaren Chips 9 ausgebildet, wobei der Chip 9 als CMOS-Sensor 10 ausgebildet ist. Der CMOS-Sensor 10 umfasst einen Bayerfilter 11 (aus Übersichtlichkeitsgründen nicht dargestellt).

Das Objektiv 5 umfasst acht Linsen 6 und zwei Planscheiben 20. Die Linsen 6 sind sphärisch ausgebildet, können jedoch im Allgemeinen auch asphärisch ausgebildet sein.

Ein Strahlengang 17 von Licht, ausgehend von der Oberflächengeometrie des Auges 2 trifft nach einer Transmission durch das Objektiv 5 auf den CMOS-Sensor 10, wobei das Licht des Auges 2 in chronologischer Reihenfolge durch eine Objektivlinse 26, ein Leuchtpunktabsehen 21, eine Meniskuslinse 27, eine objektseitige Luftlinse 6, eine objektseitige Zerstreuungslinse 28, eine Blende, eine Sammellinse 29, einen Achromat 30, eine sensorseitige Meniskuslinse 27, eine sensorseitige Zerstreuungslinse 28 und ein Schutzglas transmittiert wird.

Zwei derartige Strahlengänge 17 sind in der Darstellung gezeigt, wobei - ausgehend vom Auge 2 - der obere Strahlengang 17 von einem stärker gekrümmten Bereich der Oberflächengeometrie des Auges 2 und der untere Strahlengang 17 von einem geringer gekrümmten Bereich der Oberflächengeometrie des Auges 2 ausgeht. Durch die Transmission durch die acht Linsen 6 trifft der obere Strahlengang an einem unteren Bereich und der untere Strahlengang an einem oberen Bereich relativ zu dem unteren Bereich des CMOS-Sensors 10 auf.

Eine Planscheibe 20 umfasst das Leuchtpunktabsehen 21 und die zweite Planscheibe 20 agiert als Schutzglas für die Vorrichtung zur Umwandlung 4.

Vier Linsen 6 sind in Form zweier voneinander beabstandete Luftlinsen 12 ausgebildet, wobei eine Petzvalsumme der zwei Luftlinsen 12 negativ ist. Die zwei Luftlinsen sind bikonvex ausgebildet.

Die zwei Luftlinsen 12 umfassen jeweils einen linsenförmigen Fluideinschluss 15, wobei der linsenförmige Fluideinschluss 15 zwischen einer sensorseitigen Linse 6 und einer objektseitigen Linse 6 angeordnet ist. Die Petzvalsumme der sensorseitigen Linse 6, der objektseitigen Linse 6 und des Fluideinschlusses 15 ist negativ.

Die Luftlinse 12 ist derart ausgebildet, dass objektseitig eine Linse 6 mit einem ersten Brechungsindex und sensorseitig eine Linse 6 mit einem zweiten Brechungsindex angeordnet ist, wobei der zweite Brechungsindex höher als der erste Brechungsindex ausgebildet ist.

Fig. 3b zeigt das Auge 2, das Objektiv 5 sowie die Vorrichtung zur Umwandlung 4 gemäß dem in Fig. 3a gezeigten Ausführungsbeispiel, wobei zum oberen und unteren Strahlengang 17 jeweils zwei weitere Strahlengänge 17 gezeigt sind, welche jeweils einen kleineren respektive größeren Öffnungswinkel zu einer Längsausrichtung des

Objektivs 5 aufweisen. Trotz der unterschiedlichen Öffnungswinkel treffen gleiche Punkte auf der Oberflächengeometrie des Auges 2 nach Transmission durch das Objektiv 5 auf gleiche Punkte auf dem CMOS-Sensor 10.

Da Strahlengänge 17 gleicher Punkte des Auges 2 unterschiedlicher Öffnungswinkel auf gleiche Punkte auf der Vorrichtung zur Umwandlung 4 projiziert werden, ergibt sich eine scharfe Abbildung 16 des Auges 2 auf der Vorrichtung zur Umwandlung 4, wobei die scharfe Abbildung 16 nicht auf eine Ringzone des Auges 2 beschränkt ist.

Der Grund für die scharfe Abbildung 16 des Auges 2 auf dem CMOS-Sensor 10 ist eine konvex gekrümmte Schärfefläche 7 (aus Übersichtlichkeitsgründen nicht dargestellt) des Objektivs 5. Eine konvex gekrümmte Bildfläche 8, welche ohne Objektiv 5 mit der konvex gekrümmten Oberflächengeometrie des Auges 2 korrespondieren würde, wird durch das Objektiv 5 mit der konvex gekrümmten Schärfefläche 7 scharf auf die Vorrichtung zur Umwandlung 4 abgebildet. Eine scharfe Abbildung 16 des Auges 2 am CMOS-Sensor 10 ist das Resultat dieser konstruktiven Ausgestaltung des Objektivs 5.

Fig. 3c zeigt das Objektiv 5 gemäß Fig. 3a, wobei unterschiedliche Strahlengänge 17, ausgehend von einem Punkt auf der Oberfläche des Auges 2, welche jeweils durch das Objektiv 5 auf den gleichen Punkt auf dem CMOS-Sensor 10 abgebildet werden. Eine unscharfe Abbildung 16 wird dadurch unterbunden.

Die Objektivlinse 26, die objektseitige Meniskuslinse 27 und der Achromat 30 umfassen Flintglas. Die Sammellinse 29 und der Achromat 30 umfassen Kronglas.

Das Objektiv 5 kann auch einen oder mehrere Spiegel 42 (in der Darstellung nicht ersichtlich) umfassen, um die Strahlengänge 17 besonders begünstigend auf den CMOS-Sensor 10 zu lenken und eine scharfe Abbildung 16 des Auges 2 zu verbessern.

Ein Fluoreszenzfilter 43 ist zwischen der wenigstens einen Vorrichtung zur Umwandlung 4 und dem Auge 2 angeordnet. Der Fluoreszenzfilter wird besonders bevorzugt im Fokus einer der Linsen 6 platziert.

Zwischen der Zerstreuungslinse 28 und der Sammellinse 29 kann eine Pupillenblende angeordnet sein.

Fig. 3d zeigt das Objektiv 5 gemäß Fig. 3a, wobei ersichtlich ist, dass das Objektiv 5 und eine objektseitige Abbildung 16a (aus Übersichtlichkeitsgründen nicht dargestellt) und ein objektseitiger Strahlengang 17 im Wesentlichen telezentrisch ausgebildet sind. Das Objektiv 5, die objektseitige Abbildung 16 und der objektseitige Strahlengang 17 können auch perizentrisch ausgebildet sein.

Um über eine Ausrichtung des Auges 2 über einen längeren Zeitraum relativ zum Objektiv 5 zu fixieren, ist das Leuchtpunktabsehen 21 in dem wenigstens einen Objektiv 5, zentral auf einer optischen Achse 22 des wenigstens einen Objektivs 5 verortet, angeordnet ist. Dies ermöglicht auch eine Normierung der Abbildungen 16 des Auges 2, da die Ausrichtung des Auges 2 nach einer Unterbrechung von Aufnahmen durch das Ophthalmoskop 1 erneut in die gewünschte Position gelenkt werden kann.

Das Leuchtpunktabsehen 21 umfasst eine diffraktive Struktur 23, wobei die diffraktive Struktur 23 eine Ausdehnung im Bereich von 25 µm bis 75 µm aufweist. Das Leuchtpunktabsehen 21 ist sensorseitig durch eine (aus Übersichtlichkeitsgründen nicht dargestellte) Maske 24 abgedeckt, um die Abbildung 16 des Auges 2 auf der Vorrichtung zur Umwandlung 4 nicht zu beeinträchtigen. Die diffraktive Struktur 23 kann seitlich mit einer LED in einer beliebigen Farbe beleuchtet werden, wobei das Licht der LED in Richtung des Auges umgelenkt wird.

Eine optische Länge der Strahlengänge ist gleich. Um dies zu bewerkstelligen, ist eine optische Dichte am Rand ist erhöht und im Zentrum verringert. Diese Kompensation kann durch bikonvexe Luftlinsen 12 generiert werden.

Das Objektiv 5 ist konstruktiv so ausgestaltet, dass eine optische Transmission über das gesamte Feld homogen und besonders hoch ist.

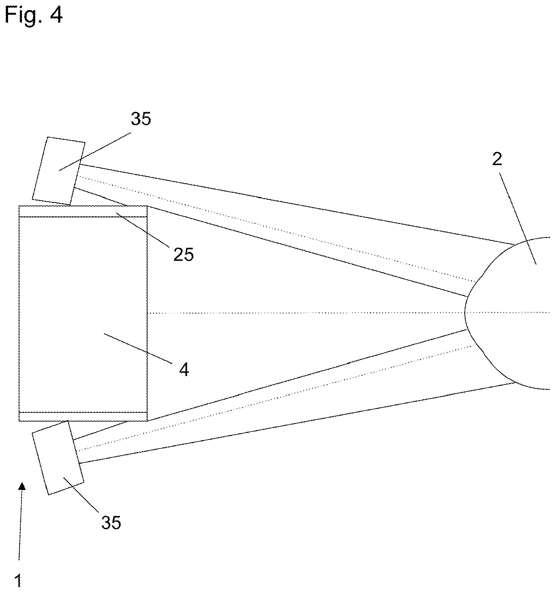

Fig. 4 zeigt ein Objektiv 5, wobei das Gehäuse 3 einen Randzylinder 25 umfasst. Der Randzylinder 25 ist geschwärzt ausgebildet. Es kann auch vorgesehen sein, dass der Randzylinder 25 nur in einem Bereich des Leuchtpunktabsehens 21 geschwärzt ausgebildet ist.

Am Gehäuse 3 ist eine Beleuchtung 35 angeordnet ist, wobei die Illumination des Auges 2 über die Beleuchtung 35 bereichsweise durch das Objektiv 5 verdeckt ist. Die Form der Beleuchtung 35 ist im Allgemeinen beliebig. Besonders bevorzugt ist eine Beleuchtung 35, welche ringförmig um das Gehäuse 3 angeordnet ist. Es können jedoch auch mehrere Beleuchtungen 35 am Gehäuse 3 angeordnet sein oder eine Beleuchtung 35 in Form einer handelsüblichen Lampe verwendet werden.

Jene Illumination des Auges 2, welche durch das Objektiv 5 verdeckt wird, würde zu ungewünschten Reflexen auf der Vorrichtung zur Umwandlung 4 führen, welche zudem eine unerwünschte Überbelichtung von Bereichen des Auges 2 führen würde. Zudem kann durch den verdeckten Bereich der Illumination eine Erweiterung der Pupille und/oder Schmerzempfinden reduziert werden. Zentrale Bereiche einer Kornea werden somit nicht direkt beleuchtet, sondern dieser Bereich der Illumination wird abgelenkt oder abgeschattet.

Die Beleuchtung 35 ist derart ausgebildet, dass das Licht der Beleuchtung 35, welches diffus bei einem Eindringen in die Kornea und eine Sklera gestreut wird, zur Abbildung 16 beiträgt.

Das Licht der Beleuchtung 35 nimmt in Richtung eines Außenbereiches des Auges 2 zu, um auf der Vorrichtung zur Umwandlung 4 eine homogene Helligkeitsverteilung zu generieren.

Die Beleuchtung 35 kann in Form einer Weißlichtbeleuchtung, beispielsweise mit einem sonnenlichtähnlichen Weißton mit einer charakteristischen Farbtemperatur zwischen 5000 K und 6000 K und/oder einem Farbwiedergabeindex von zumindest 95 % vorliegen. Die Beleuchtung 35 kann polychromatisches Licht umfassen. Die Beleuchtung 35 kann auch als fluoreszente Beleuchtung, beispielsweise mit einem Wellenlängenbereich zwischen 450 nm und 510 nm und/oder zwischen 750 nm und 780 nm, ausgebildet sein.

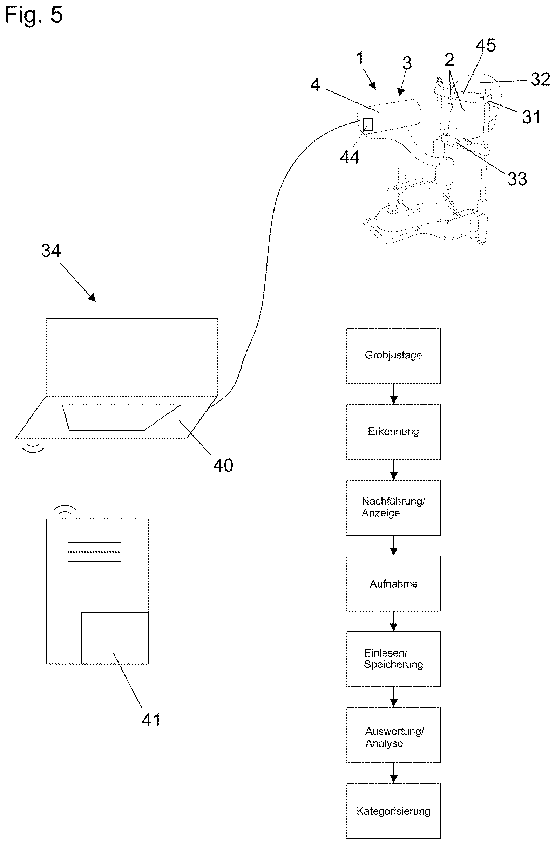

Fig. 5 zeigt ein Ophthalmoskop 1, wobei die Vorrichtung zur Umwandlung 4 des Ophthalmoskops 1 in direkter Datenverbindung mit einem Computer 34 steht. Der Computer 34 steht in signalübertragender Datenverbindung mit einer Datenbank auf einem dezentralen Rechner. Die Datenbank kann jedoch auch direkt auf dem Computer 34 vorhanden sein.

Ein Flussdiagramm beschreibt ein Verfahren zur Untersuchung von Augen 2, umfassend die Schritte
- Grobjustage eines Patienten in einer Haltevorrichtung 31, wobei eine Genauigkeit der Grobjustage unterhalb 3 mm besonders günstig für scharfe Abbildungen des Auges 2 auf der Vorrichtung zur Umwandlung 4 ist,
- Erkennung einer Pupille und/oder eines Scheitels einer Kornea des Auges 2 über ein Bildverarbeitungsprogramm, wobei das Bildverarbeitungsprogramm einen Autofukusalgorithmus zur Unterstützung der Erkennung aufweisen kann und
- Aufnahme von zumindest einer Abbildung 16 des Auges 2 durch das Ophthalmoskop 1, wobei die zumindest eine Abbildung 16 des Auges 2 auf der Vorrichtung zur Umwandlung 4 aufgenommen wird.

Weiters sind gemäß diesem aufgezeigten Ausführungsbeispiel des Verfahrens weitere Schritte vorgesehen:
- Bewegungen des Auges 2 unterhalb von 3 mm können durch eine automatisierte Nachführung nachgeführt werden und/oder eine Öffnung eines Augenlides kann über das Bildverarbeitungsprogramm erkannt werden - bei einer Öffnung des Auges 2 von zumindest 12 mm, vorzugsweise zumindest 16 mm, kann eine Anzeige an einem Statusbildschirm 44 angezeigt werden,
- die zumindest eine Abbildung 16 des Auges 2 auf der Vorrichtung zur Umwandlung 4 wird in den Computer 34 eingelesen und gespeichert,
- die zumindest eine Abbildung 16 des Auges 2 wird hinsichtlich augenspezifischer Charakteristika ausgewertet und analysiert und
- die zumindest eine Abbildung 16 des Auges 2 wird am Computer 34 zur Standardisierung und Normierung kategorisiert.

Ein Computerprogrammprodukt ist am Computer 34 vorhanden, welches Befehle umfasst, die bei der Ausführung durch eine Recheneinheit 40 diese veranlassen, für ein Ophthalmoskop 1 zur Untersuchung von Augen 2
- aus einer Speichereinheit 41, welche mit der Recheneinheit 40 in einer Datenverbindung steht, die zumindest eine Abbildung 16 des Auges 2 zu klassifizieren, wobei eine Klassifikation basierend auf einer Blutgefäßstruktur 46 oder einer Hornhautstruktur oder einer Narbenstruktur oder einer Augenhöhlenstruktur generiert wird,
- wobei eine Bildüberlagerung mit automatischer Blutgefäßerkennung oder automatischer Limbuserkennung zur Kontrolle der Klassifikation herangezogen wird,
- wobei eine halbautomatische Läsionserkennung oder bidirektionale Messungen vorgenommen wird, und
- wobei standardisierte Abbildungen 16 wenigstens einer Iris in der Speichereinheit 41 abgespeichert werden.

Das Computerprogrammprodukt kann auch abhängig von einer Farbe zweier Iris eine Farbe einer Kontaktlinse und einer künstlichen Iris auswählen, wobei die Farbe der Kontaktlinse und der künstliche Iris an eine der beiden Iris angepasst ist.

Weiters kann das Computerprogrammprodukt die Blutgefäßstruktur 46 automatisch erkennen und hierbei beispielsweise künstliche Intelligenz verwenden. Auch eine Quantifizierung von Veränderungen der Blutgefäßstruktur 46 und/oder der Hornhautstruktur kann über die Bildüberlagerung berechnet werden.

Des Weiteren ist es möglich, eine Rotpixeldichtemessung vorzunehmen, wobei die Rotpixelmessung einen gesamten Rotanteil der wenigstens einen Abbildung 16 integriert und/oder einen Flächenanteil der Blutgefäßstruktur 46 an der wenigstens einen Abbildung 16 errechnet.

Das Computerprogrammprodukt umfasst eine automatische Läsionserkennung des Auges 2 und kann eine Öffnung des Auges 2 von zumindest 12 mm erkennen, wobei bei einer Öffnung von unter 12 mm eine elektronische Information an den wenigstens einen Statusbildschirm 44 des Ophthalmoskops 1 übermittelt wird.

Das Ophthalmoskop 1 kann in einer Stand-Alone-Ausführung oder als Add-On-Ausführung für eine handelsübliche Spaltlampe 36 verwendet werden.

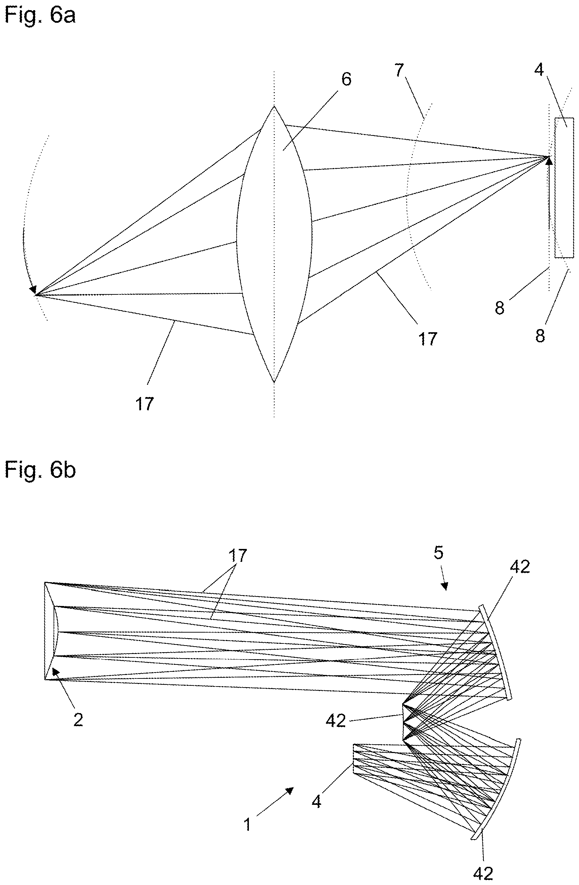

Fig. 6a zeigt ein konkav gekrümmtes Objekt mit einer konkav gekrümmten Objektfläche und eine Vorrichtung zur Umwandlung 4. Strahlengänge 17 visualisieren die Transmission von Licht durch eine Linse 6, ausgehend vom konkav gekrümmten Objekt in Richtung der Vorrichtung zur Umwandlung 4.

Ohne Linse 6 würde die konkav gekrümmte Objektfläche auf eine konkav gekrümmte Bildlfäche 8 (durch eine strichlierte Linie angedeutet) abgebildet werden. Durch die Linse 6 wird eine Abbildung 16 auf eine planare Vorrichtung zur Umwandlung 4 ermöglicht, wobei die Bildfläche 8 ebenfalls planar (durch eine strichlierte Linie angedeutet) ausgebildet ist. Die konkav gekrümmte Bildfläche 8 ist somit durch die Linse 6 scharf auf die Vorrichtung zur Umwandlung 4 abbildbar. Bei einem konvex gekrümmten Objekt würde sich bei dieser Linse 6 eine Konvexität noch verstärken, wodurch eine scharfe Abbildung 16 auf die planare Vorrichtung zur Umwandlung 4 nicht möglich wäre.

Die Schärfefläche 7 ist rechts der Linse 6 definiert, wobei in der Darstellung die Schärfefläche 7 durch eine strichlierte Linie in der Bildebene angedeutet ist. Die Schärfefläche 7 ist konkav ausgebildet, wobei erfindungsgemäß eine konvexe Schärfefläche 7 vorgesehen ist.

Fig. 6b zeigt Strahlengänge 17 zwischen einem Auge 2 und einer Vorrichtung zur Umwandlung 4, wobei die Strahlengänge 17 durch drei Spiegel 42 auf die Vorrichtung zur Umwandlung umgelenkt werden.

Die Spiegel 42 sind hierbei als Freiformspiegel ausgebildet. Die Spiegel 42 können jedoch ebenso anderwärtige Spiegelarten umfassen.

Das Objektiv 5 des Ophthalmoskops weist eine gekrümmte Schärfefläche 7 (aus Übersichtlichkeitsgründen nicht dargestellt) auf, um das Auge 2 scharf auf die Vorrichtung zur Umwandlung 4 abbilden zu können.

Eine Verwendung des Objektivs 5 am Auge 2 in Kombination mit wenigstens einer Linse 6 ist ebenfalls möglich.

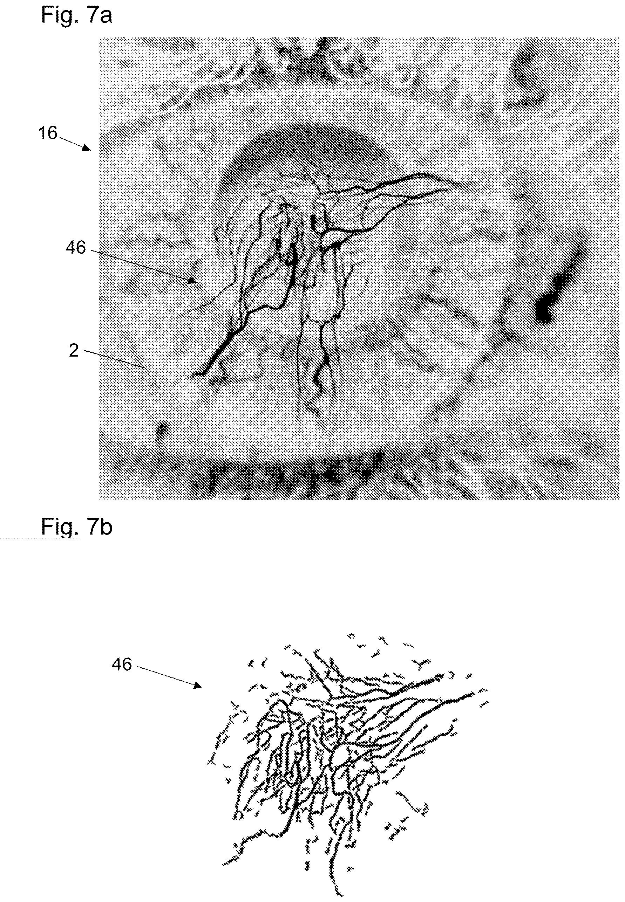

Fig. 7a zeigt eine Abbildung 16 eines menschlichen Auges 2. Eine Blutgefäßstruktur 46 ist zu erkennen, wobei die Blutgefäßstruktur 46 durch eine rötliche Farbe identifiziert werden kann.

Fig. 7b zeigt eine Blutgefäßstruktur 46, welche über die Abbildung 16 des Auges 2 nach Fig. 7a durch das Computerprogrammprodukt mit der automatischen Blutgefäßerkennung generiert wurde.

Anhand der Blutgefäßstruktur 46 sind eine Analyse, eine Kategorisierung und/oder eine Rotpixeldichtemessung der Abbildung 16 des Auges vornehmbar.

## Patentansprüche

1. Ophthalmoskop (1) zur Untersuchung einer sichtbaren Oberfläche von Augen (2) umfassend
- ein Gehäuse (3),
- wenigstens eine Vorrichtung zur Umwandlung (4) von Licht in ein elektrisches Signal in Form eines planaren Chips (9) und
- wenigstens ein Objektiv (5), wobei das wenigstens eine Objektiv (5) wenigstens eine Linse (6) und/oder wenigstens einen Spiegel (42) umfasst,
**dadurch gekennzeichnet, dass** das wenigstens eine Objektiv (5) eine konvex gekrümmte Schärfefläche (7) als jene Fläche, welche bei einer Projektion einer Objektebene durch das Objektiv (5) generiert wird, um eine Bildfläche einer Objektfläche auf der Vorrichtung zur Umwandlung geändert darzustellen aufweist, wobei eine konvex gekrümmte Bildfläche (8) eines Auges (2) durch das wenigstens eine Objektiv (5) scharf auf die wenigstens eine Vorrichtung zur Umwandlung (4) abbildbar ist, wobei der gesamte sichtbare Bereich des Auges (2) scharf auf die wenigstens eine Vorrichtung zur Umwandlung (4) abbildbar ist und wobei das Ophthalmoskop (1) eine gepulste Beleuchtung (35) umfasst.

2. Ophthalmoskop (1) nach Anspruch 1, wobei
- die Beleuchtung (35) derart ausgebildet ist, dass das Licht der Beleuchtung (35), welches diffus bei einem Eindringen in die Kornea und eine Sklera gestreut wird, zur Abbildung (16) beiträgt, und/oder
- eine Auflösung der Abbildung des Auges (2) dergestalt ist, dass ein Kontrast eines Linienpaares zwischen getrennten, benachbarten Pixeln auf der wenigstens einen Vorrichtung zur Umwandlung (4) von zumindest 20%, vorzugsweise zumindest 30%, besteht, und/oder
- die wenigstens eine Beleuchtung (35) mit einem Pulsweitenmodulationssignal ansteuerbar oder angesteuert ist.

3. Ophthalmoskop (1) nach Anspruch 1 oder 2, wobei die wenigstens eine Vorrichtung zur Umwandlung (4)
- als CMOS (10) ausgebildet ist, und/oder
- wenigstens einen Bayerfilter (11) umfasst.

4. Ophthalmoskop (1) nach einem der vorangehenden Ansprüche, wobei eine Linsenanordnung mit negativer Petzvalsumme zur Abbildung der konvex gekrümmten Bildfläche (8) des Auges (2) vorgesehen ist und/oder wenigstens zwei Linsen (6) in Form wenigstens einer Luftlinse (12) ausgebildet sind, wobei eine Petzvalsumme der wenigstens einen Luftlinse (12) negativ ist, wobei vorzugsweise innerhalb der wenigstens einen Luftlinse (12) ein Gasgemisch mit einem Brechungsindex kleiner 1.3, bevorzugt kleiner 1.1 angeordnet ist, wobei vorzugsweise vorgesehen ist, dass das Gasgemisch zumindest ein Edelgas, besonders bevorzugt Argon, Stickstoff und/oder Luft enthält, besonders bevorzugt in Form des Edelgases, von Stickstoff oder von Luft vorliegt.

5. Ophthalmoskop (1) nach einem der vorangehenden Ansprüche, wobei zwischen einer sensorseitigen Linse (6) und einer objektseitigen Linse (6) ein linsenförmiger Fluideinschluss (15) angeordnet ist, wobei die Petzvalsumme der sensorseitigen Linse (6), der objektseitigen Linse (6) und des Fluideinschlusses (15) negativ ist.

6. Ophthalmoskop (1) nach Anspruch 4 oder 5, wobei
- die wenigstens eine Luftlinse (12) derart ausgebildet ist, dass objektseitig eine Linse (6) mit einem ersten Brechungsindex und sensorseitig eine Linse (6) mit einem zweiten Brechungsindex angeordnet ist, wobei der zweite Brechungsindex höher als der erste Brechungsindex ist und/oder die wenigstens eine Luftlinse (12) bikonvex ausgebildet ist, und/oder
- genau zwei voneinander beabstandete Luftlinsen (12) vorhanden sind.

7. Ophthalmoskop (1) nach einem der vorangehenden Ansprüche, wobei
- das wenigstens eine Objektiv (5) und/oder eine objektseitige Abbildung (16a) und/oder ein objektseitiger Strahlengang (17) im Wesentlichen telezentrisch, vorzugsweise perizentrisch, ausgebildet ist,
- eine Abbildung (16) auf der wenigstens einen Vorrichtung zur Umwandlung (4) farbig dargestellt ist,
- eine sensorseitige numerische Apertur des wenigstens einen Objektivs (5) im Bereich von 0,04 bis 0,1, vorzugsweise im Bereich von 0,06 bis 0,08, liegt,
- das wenigstens eine Objektiv (5) eine Blende aufweist, wobei die Blende einen Radius im Bereich von 2,5 mm bis 3,5 mm, vorzugsweise im Bereich von 2,9 bis 3,3, aufweist,
- das Ophthalmoskop (1) genau acht Linsen (6) und/oder genau zwei Planscheiben (20) umfasst,
- wenigstens eine Linse (6) asphärisch ausgebildet ist,
- wenigstens ein Leuchtpunktabsehen (21) in dem wenigstens einen Objektiv (5), vorzugsweise zentral auf einer optischen Achse (22) des wenigstens einen Objektivs (5) verortet, angeordnet ist, wobei vorzugsweise vorgesehen ist, dass das wenigstens eine Leuchtpunktabsehen (21) wenigstens eine diffraktive Struktur (23) umfasst, wobei die wenigstens eine diffraktive Struktur (23) eine Ausdehnung im Bereich von 25 µm bis 75 pm, vorzugsweise im Bereich von 40 µm bis 55 µm, aufweist und/oder das wenigstens eine Leuchtpunktabsehen (21) sensorseitig durch eine Maske (24) abgedeckt ist, und/oder
- das Gehäuse (3) wenigstens einen Randzylinder (25) umfasst, wobei der wenigstens eine Randzylinder (25), vorzugsweise im Bereich des wenigstens einen Leuchtpunktabsehens (21), geschwärzt ausgebildet ist.

8. Ophthalmoskop (1) nach Anspruch 7, wobei das Licht des Auges (2) in chronologischer Reihenfolge durch eine Objektivlinse (26), das Leuchtpunktabsehen (21), eine Meniskuslinse (27), eine objektseitige Luftlinse (14), eine objektseitige Zerstreuungslinse (28), die Blende, eine Sammellinse (29), einen Achromat (30), eine sensorseitige Meniskuslinse (27), eine sensorseitige Zerstreuungslinse (28) und ein Schutzglas transmittierbar ist, wobei das Licht des Auges (2) anschließend auf die wenigstens eine Vorrichtung zur Umwandlung (4) treffbar ist, wobei vorzugsweise vorgesehen ist, dass die Objektivlinse (26) und/oder die objektseitige Meniskuslinse (27) und/oder der Achromat (30) Flintglas umfassen und/oder die Sammellinse (29) und/oder der Achromat (30) Kronglas umfassen.

9. Ophthalmoskop (1) nach einem der vorangehenden Ansprüche, wobei
- wenigstens eine Haltevorrichtung (31) für einen Kopf (32), vorzugsweise mit einer Kinnauflage (33) und/oder einer Stirnstütze (45), vorgesehen ist, wobei vorzugsweise vorgesehen ist, dass die Haltevorrichtung (31) von der wenigstens einen Vorrichtung zur Umwandlung (4) im Bereich von 30 mm bis 200 mm, vorzugsweise im Bereich von 40 mm bis 100 mm, beabstandet ist,
- die wenigstens eine Vorrichtung zur Umwandlung (4) in Datenverbindung mit wenigstens einem Computer (34) bringbar ist, wobei der wenigstens eine Computer (34) in Verbindung mit einer Datenbank bringbar ist,
- am Gehäuse (3) wenigstens eine Beleuchtung (35) angeordnet ist, wobei die Illumination des Auges (2) über die wenigstens eine Beleuchtung (35) bereichsweise durch das wenigstens eine Objektiv (5) verdeckt ist, wobei vorzugsweise vorgesehen ist, dass die wenigstens eine Beleuchtung (35) in Form einer Weißlichtbeleuchtung, vorzugsweise mit einem sonnenlichtähnlichen Weißton mit einer charakteristischen Farbtemperatur zwischen 5000 K und 6000 K und/oder einem Farbwiedergabeindex von zumindest 95 %, und/oder einer fluoreszenten Beleuchtung ausgestaltet ist und/oder die fluoreszente Beleuchtung einen Wellenlängenbereich zwischen 450 nm und 510 nm und/oder zwischen 750 nm und 780 nm aufweist,
- wenigstens ein Fluoreszenzfilter (43) zwischen der wenigstens einen Vorrichtung zur Umwandlung (4) und dem Auge (2) angeordnet ist, und/oder
- das Ophthalmoskop (1) wenigstens einen Statusbildschirm (44) umfasst, wobei auf dem wenigstens einen Statusbildschirm (44) wenigstens eine elektronische Information visualisierbar ist.

10. Ophthalmoskop (1) nach einem der Ansprüche 1 bis 9 mit einer Spaltlampe (36).

11. Verwendung eines Ophthalmoskops (1) nach einem der Ansprüche 1 bis 10 am menschlichen Auge (2).

12. Verfahren zur Untersuchung von Augen (2), umfassend die Schritte
- Grobjustage eines Patienten in einer Haltevorrichtung (31), insbesondere mit einer Genauigkeit unterhalb 3 mm,
- Erkennung einer Pupille und/oder eines Scheitels einer Kornea des Auges (2) über ein Bildverarbeitungsprogramm, wobei insbesondere vorgesehen ist, dass das Bildverarbeitungsprogramm einen Autofukusalgorithmus aufweist,
- Aufnahme von wenigstens einer Abbildung (16) des Auges (2) durch ein Ophthalmoskop (1) gemäß einem der Ansprüche 1 bis 10, wobei die wenigstens eine Abbildung (16) des Auges (2) auf wenigstens einer Vorrichtung zur Umwandlung (4) aufgenommen wird.

13. Verfahren nach Anspruch 12, wobei
- Bewegungen des Auges (2) unterhalb von 3 mm durch eine automatisierte Nachführung nachgeführt werden und/oder eine Öffnung eines Augenlides über das Bildverarbeitungsprogramm erkannt wird, wobei vorzugsweise vorgesehen ist, dass bei einer Öffnung unter 12 mm, vorzugsweise unter 16 mm, eine Anzeige an wenigstens einem Statusbildschirm (44) angezeigt wird,
- die wenigstens eine Abbildung (16) des Auges (2) auf der wenigstens einen Vorrichtung zur Umwandlung (4) in wenigstens einen Computer (34), vorzugsweise in eine Datenbank auf dem wenigstens einen Computer (34), eingelesen und/oder gespeichert wird,
- die wenigstens eine Abbildung (16) des Auges (2) hinsichtlich augenspezifischer Charakteristika ausgewertet und/oder analysiert wird,
- die wenigstens eine Abbildung (16) des Auges (2) am wenigstens einen Computer (34) zur Standardisierung und/oder Normierung kategorisiert wird,
- das Auge (2) eine Kontaktlinse und eine mit Fluoreszenzfarbstoff angereicherte Tränenflüssigkeit aufweist, wobei die Tränenflüssigkeit mit einer fluoreszenten Beleuchtung (35) angestrahlt wird, die Tränenflüssigkeit Licht in einem Wellenlängenbereich zwischen 515 nm und 530 nm und/oder zwischen 825 nm und 835 nm emittiert und eine Verteilung des emittierten Lichtes der Tränenflüssigkeit zwischen der Kontaktlinse und dem Auge (2) auf der wenigstens einen Vorrichtung zur Umwandlung (4) aufgenommen wird, und/oder
- das Auge (2) wenigstens einen Fluoreszenzfarbstoff umfasst und eine Blutgefäßstruktur (46) und/oder eine Lymphgefäßstruktur und/oder ein Hornhautepithel des Auges (2), vorzugsweise über den wenigstens einen Fluoreszenzfilter (43), auf der wenigstens einen Vorrichtung zur Umwandlung (4) abgebildet und/oder auf dem wenigstens einen Computer (34) gespeichert wird, wobei vorzugsweise vorgesehen ist, dass der Fluoreszenzfarbstoff Fluoreszein und/oder Indizyaningrün umfasst.

14. Ophthalmoskop nach einem der Ansprüche 1 bis 10 mit einem Computerprogrammprodukt, umfassend Befehle, die bei der Ausführung durch eine Recheneinheit (40) diese veranlassen, für das Ophthalmoskop (1) nach einem der Ansprüche 1 bis 10 zur Untersuchung von Augen (2) aus einer Speichereinheit (41), welche mit der Recheneinheit (40) in einer Datenverbindung steht oder in eine solche bringbar ist, wenigstens eine Abbildung (16) eines Auges (2) zu klassifizieren, wobei eine Klassifikation basierend auf einer Blutgefäßstruktur (46) und/oder einer Hornhautstruktur und/oder einer Narbenstruktur und/oder einer Augenhöhlenstruktur generiert wird.

15. Ophtalmoskop nach Anspruch 14, wobei durch das Computerprogrammprodukt
- eine Bildüberlagerung mit automatischer Blutgefäßerkennung und/oder automatischer Limbuserkennung zur Kontrolle der Klassifikation heranziehbar ist,
- eine halbautomatische Läsionserkennung und/oder bidirektionale Messungen vornehmbar sind,
- standardisierte Abbildungen (16) wenigstens einer Iris in der Speichereinheit (41) abgespeichert werden, wobei vorzugsweise vorgesehen ist, dass abhängig von einer Farbe zweier Iris eine Farbe einer Kontaktlinse und/oder einer künstlichen Iris ausgewählt wird, wobei die Farbe der Kontaktlinse und/oder der künstlichen Iris an eine der beiden Iris angepasst ist,
- die Blutgefäßstruktur (46), vorzugsweise über künstliche Intelligenz, automatisch erkannt wird,
- über die Bildüberlagerung eine Quantifizierung von Veränderungen der Blutgefäßstruktur (46) und/oder der Hornhautstruktur berechnet wird,
- eine Rotpixeldichtemessung vorgenommen wird, wobei die Rotpixelmessung einen gesamten Rotanteil der wenigstens einen Abbildung (16) integriert und/oder einen Flächenanteil der Blutgefäßstruktur (46) an der wenigstens einen Abbildung (16) errechnet, und/oder
- eine Öffnung des Auges (2) von zumindest 12 mm, vorzugsweise zumindest 16 mm, erkannt wird und bei einer Öffnung unter 12 mm, vorzugsweise unter 16 mm, eine elektronische Information an den wenigstens einen Statusbildschirm (44) übermittelt wird,
und/oder das Computerprogrammprodukt eine automatische Läsionserkennung des Auges (2) umfasst.

## Claims

1. An ophthalmoscope (1) for examining a visible surface of eyes (2), comprising
- a housing (3),
- at least one device for conversion (4) for converting light into an electrical signal in the form of a planar chip (9), and
- at least one objective (5), wherein the at least one objective (5) comprises at least one lens (6) and/or at least one mirror (42),
**characterized in that** the at least one objective (5) has a convexly curved focus surface (7) as that surface that is generated by the objective (5) when projecting an object surface in order to display an image surface of an object surface in a modified version, wherein a convexly curved image surface (8) of an eye (2) can be sharply imaged onto the at least one device for conversion (4) by means of the at least one objective (5), wherein the entire visible area of the eye (2) can be sharply imaged on the at least one device for conversion (4) and wherein the ophthalmoscope (1) comprises a pulsed illumination (35).

2. The ophthalmoscope (1) according to claim 1, wherein
- the illumination (35) is designed in such way that the light of the illumination (35) diffused when penetrating the cornea and a sclera contributes to the image (16), and/or
- a resolution of the image of the eye (2) is displayed that a contrast of a line pair of at least 20 %, preferably 30 %, exists between separated, adjoining pixels on the at least one device for conversion (4), and/or
- the at least one illumination (35) is or can be actuated with a pulse-width modulation signal.

3. The ophthalmoscope (1) according to claim 1 or 2, wherein the at least one device for conversion (4)
- is formed as CMOS (10), and/or
- comprises at least one Bayer filter (11).

4. The ophthalmoscope (1) according to one of the preceding claims, wherein a lens arrangement which has a negative Petzval sum is provided to image the convexly curved image surface (8) of the eye (2), and/or at least two lenses (6) are formed in the shape of at least one air lens (12), wherein a Petzval sum of the at least one air lens (12) is negative, wherein a gas mixture with a refractive index smaller than 1.3, preferably smaller than 1.1, is arranged preferably within the at least one air lens (12), wherein it is preferably provided that the gas mixture contains at least one noble gas, particularly preferred argon, nitrogen and/or air, particularly preferably in the form of the noble gas nitrogen or air.

5. The ophthalmoscope (1) according to one of the preceding claims, wherein a lens-shaped fluid confinement (15) is arranged between a sensor-sided lens (6) and an object-sided lens (6), wherein the Petzval sum of the sensor-sided lens (6), the object-sided lens (6) and the fluid confinement (15) is negative.

6. The ophthalmoscope (1) according to claims 4 or 5, wherein
- the at least one air lens (12) is designed such that a lens (6) with a first refractive index is arranged on the object side, and a lens (6) with a second refractive index is arranged on the sensor side, wherein the second refractive index is higher than the first refractive index, and/or the at least one air lens (12) is designed in a biconvex manner, and/or
- exactly two spaced-apart air lenses (12) are present.

7. The ophthalmoscope (1) according to one of the preceding claims, wherein
- the at least one objective (5) and/or an object-sided image (16a) and/or an object-sided ray path (17) is designed to be essentially telecentric, preferably pericentric,
- an image (16) on the at least one device for conversion (4) is shown in color,
- a sensor-sided numerical aperture of the at least one objective (5) lies in the range from 0.04 to 0.1, preferably in the range of 0.06 to 0.08,
- the at least one objective (5) has an aperture, wherein the aperture has a radius in the range of 2.5 mm to 3.5 mm, preferably in the range of 2.9 mm to 3.3 mm,
- the ophthalmoscope (1) comprises exactly eight lenses (6) and/or exactly two planar discs (20),
- at least one lens (6) is designed to be aspherical,
- at least one illuminated dot reticle (21) is arranged in the at least one objective (5), preferably centrally located on an optical axis (22) of the at least one objective (5), wherein it is preferably provided that the at least one illuminated dot reticle (21) comprises at least one diffractive structure (23), wherein the at least one diffractive structure (23) has an extent in the range from 25 µm to 75 pm, preferably in the range of 40 µm to 55 µm, and/or the at least one illuminated dot reticle (21) is covered on the sensor side by a mask (24), and/or
- the housing (3) comprises at least one peripheral cylinder (25), wherein the at least one peripheral cylinder (25) is designed blackened, preferably in the portion of the at least one illuminated dot reticle (21).

8. The ophthalmoscope (1) according to claim 7, wherein the light of the eye (2) can be transmitted in chronological order through an objective lens (26), the illuminated spot reticle (21), a meniscus lens (27), an object-sided air lens (14), an object-sided diverging lens (28), the aperture , a converging lens (29), an achromat (30), a sensor-sided meniscus lens (27), a sensor-sided diverging lens (28), and a protective glass (31), wherein the light of the eye (2) can subsequently be impinged upon the at least one device for conversion (4), wherein it is preferably provided that the object lens (26) and/or the object-sided meniscus lens (27) and/or the achromat (30) comprise flint glass (32), and/or the converging lens (29) and/or the achromat (30) comprise crown glass (30).

9. The ophthalmoscope (1) according to one of the preceding claims, wherein
- at least one holding device (31) for a head (32) is provided , preferably with the holding device (31) having a chin rest (33) and/or a forehead support (45), wherein it is preferably provided that the holding device (31) is spaced from the at least one device for conversion (4) in the range from 30 mm to 200 mm, preferably in the range of 40 mm to 100 mm,
- the at least one device for conversion (4) can be brought into data connection with at least one computer (34), wherein the at least one computer (34) can be brought into connection with a database,
- at least one illumination (35) is arranged on the housing (3), wherein the illumination of the eye (2) via the at least one illumination (35) is partially covered by the at least one objective (5), wherein it is preferably provided that the at least one illumination (35) is designed in the form of a white light illumination, preferably with a sunlight-like white tone with a characteristic color temperature between 5000 K and 6000 K and/or a color rendering index of at least 95% and/or a fluorescent illumination, and/or the fluorescent illumination has a wavelength range between 450 nm and 510 nm and/or between 750 nm and 780 nm,
- at least one fluorescence filter (43) is arranged between the at least one device for conversion (4) and the eye (2), and/or
- the ophthalmoscope (1) comprises at least one status screen (44), wherein at least one electronic information can be visualized on the at least one status screen (44).

10. The ophthalmoscope (1) according to one of claims 1 to 9 with a slit lamp (36).

11. A use of an ophthalmoscope (1) according to one of claims 1 to 10 on the human eye (2).

12. A method for examining eyes (2), comprising the following steps:
- rough adjustment of a patient in a holding device (31), in particular with an accuracy below 3 mm,
- detection of a pupil and/or a vertex of a cornea of the eye (2) via an image processing program, wherein it is particularly provided that the image processing program comprises an autofocus algorithm,
- capturing at least one image (16) of the eye (2) by an ophthalmoscope (1) according to one of claims 1 to 10, wherein the at least one image (16) of the eye (2) is captured on at least one device for conversion (4) .

13. The method according to claim 12, wherein
- movements of the eye (2) below 3 mm are tracked by automated tracking, and/or an opening of an eyelid is detected via the image processing program, wherein it is preferably provided that when an opening is below 12 mm, preferably below 16 mm, an indication is displayed on at least one status screen (44),
- the at least one image (16) of the eye (2) on the at least one device for conversion (4) is read into and/or saved in at least one computer (34), preferably in a database on the at least one computer (34),
- the at least one image (16) of the eye (2) is evaluated and/or analyzed with regard to eye-specific characteristics,
- the at least one image (16) of the eye (2) is categorized at the at least one computer (34) for standardization and/or norming, or
- the eye (2) comprises a contact lens and a tear fluid enriched with fluorescent dye, wherein the tear fluid is illuminated with fluorescent illumination (35), the tear fluid emits light in a wavelength range between 515 nm and 530 nm and/or between 825 nm and 835 nm, and a distribution of the emitted light of the tear fluid between the contact lens and the eye (2) is recorded on the at least one device for conversion (4), and/or
- the eye (2) comprises at least one fluorescent dye and a blood vessel structure (46) and/or a lymphatic vessel structure and/or a corneal epithelium of the eye (2), preferably via at least one fluorescence filter (43), is imaged on the at least one device for conversion (4) and/or saved on the at least one computer (34), wherein it is preferably provided that the fluorescent dye comprises fluorescein or indicyanine green.

14. The ophthalmoscope (1) according to one of claims 1 to 10 having a computer program product comprising commands, which, when carried out by a computing unit (40), cause the computing unit (40) to classify from a memory unit (41), which is or can be brought in data connection with the computing unit (40), at least one image (16) of an eye (2) for an ophthalmoscope 1 according to one of claims 1 to 10 for examining eyes (2), wherein a classification is generated, based on a blood vessel structure (46) and/or a corneal structure and/or a scar structure and/or an eye socket structure.

15. The ophthalmoscope (1) according to claim 14, wherein with the computer program product
- an image overlay with automatic blood vessel detection and/or automatic limbus detection can be used to control the classification,
- a semi-automatic lesion detection and/or bidirectional measurements can be carried out,
- standardized images (16) of at least one iris are saved in the memory unit (41), wherein it is preferably provided that a color of a contact lens and/or an artificial iris is selected depending on a color of two iris, wherein the color of the contact lens and/or the artificial iris is matched to one of the two iris,
- the blood vessel structure (46) is automatically detected, preferably via artificial intelligence,
- a quantification of changes in the blood vessel structure (46) and/or the corneal structure is calculated via the image overlay,
- a red pixel density measurement is carried out, wherein the red pixel measurement integrates a total red portion of the at least one image (16), and/or calculates an area portion of the blood vessel structure (46) at the at least one image (16), and/or
- an opening of the eye (2) of at least 12 mm, preferably at least 16 mm, is detected and when the opening is less than 12 mm, preferably less than 16 mm, an electronic information is transmitted to the at least one status screen (44), and/or the computer program product comprises an automatic lesion detection of the eye (2).

## Revendications

1. Ophtalmoscope (1) pour l'examen d'une surface visible des yeux (2) comprenant
- un boîtier (3),
- au moins un dispositif de conversion (4) de lumière en un signal électrique sous la forme d'une puce planaire (9) et
- au moins un objectif (5), dans lequel l'au moins un objectif (5) comprend au moins une lentille (6) et/ou au moins un miroir (42),
**caractérisé en ce que** l'au moins objectif (5) présente une surface de mise au point (7) incurvée de manière convexe comme la surface qui est générée lors d'une projection d'un plan d'objet par l'objectif (5) afin de représenter de manière modifiée une surface d'image d'une surface d'objet sur le dispositif de conversion, dans lequel une surface d'image (8) incurvée de manière convexe d'un œil (2) peut être imagée par l'au moins un objectif (5) de manière nette sur l'au moins un dispositif de conversion (4), dans lequel la zone visible entière de l'œil (2) peut être imagée de manière nette sur l'au moins un dispositif de conversion (4) et dans lequel l'ophtalmoscope (1) comprend un éclairage (35) pulsé.

2. Ophtalmoscope (1) selon la revendication 1, dans lequel
- l'éclairage (35) est réalisé de telle manière que la lumière de l'éclairage (35) qui est dispersée de manière diffuse lors d'une pénétration dans la cornée et une sclère, contribue à la reproduction (16), et/ou
- une résolution de l'image de l'œil (2) est telle qu'il existe un contraste d'une paire de lignes entre des pixels contigus séparés sur l'au moins un dispositif de conversion (4) d'au moins 20 %, de préférence d'au moins 30 %, et/ou
- l'au moins un éclairage (35) est ou peut être commandé avec un signal de modulation d'impulsions en largeur.

3. Ophtalmoscope (1) selon la revendication 1 ou 2, dans lequel l'au moins un dispositif de conversion (4)
- est réalisé comme CMOS (10) et/ou
- comprend au moins un filtre Bayer (11).

4. Ophtalmoscope (1) selon l'une quelconque des revendications précédentes, dans lequel un ensemble de lentille est prévu avec la somme de Petzval négative pour la reproduction de la surface d'image (8) incurvée de manière convexe de l'œil (2) et/ou au moins deux lentilles (6) sont réalisées sous la forme d'au moins une lentille d'air (12), dans lequel une somme de Petzval de l'au moins une lentille d'air (12) est négative, dans lequel un mélange de gaz avec un indice de réfaction inférieur à 1,3, de préférence inférieur à 1,1 est de préférence agencé à l'intérieur de l'au moins une lentille d'air (12), dans lequel il est de préférence prévu que le mélange de gaz contienne au moins un gaz rare, de manière particulièrement préférée de l'argon, de l'azote et/ou de l'air, se présente de manière particulièrement préférée sous la forme de gaz rare, d'azote ou d'air.

5. Ophtalmoscope (1) selon l'une quelconque des revendications précédentes, dans lequel une inclusion de fluide (15) en forme de lentille est agencée entre une lentille (6) côté capteur et une lentille (6) côté objet, dans lequel la somme de Petzval de la lentille (6) côté capteur, de la lentille (6) côté objet et de l'inclusion de fluide (15) est négative.

6. Ophtalmoscope (1) selon la revendication 4 ou 5, dans lequel
- l'au moins une lentille d'air (12) est réalisée de telle manière qu'une lentille (6) avec un premier indice de réfraction soit agencée côté objet et une lentille (6) avec un deuxième indice de réfraction soit agencée côté capteur, dans lequel le deuxième indice de réfraction est supérieur au premier indice de réfraction et/ou l'au moins une lentille d'air (12) est réalisée de manière biconvexe, et/ou
- précisément deux lentilles d'air (12) espacées l'une de l'autre sont présentes.

7. Ophtalmoscope (1) selon l'une quelconque des revendications précédentes, dans lequel
- l'au moins un objectif (5) et/ou une reproduction (16a) côté objet et/ou une trajectoire de faisceau (17) côté objet est réalisé(e) sensiblement de manière télécentrique, de préférence péricentrique,
- une reproduction (16) est représentée en couleur sur l'au moins un dispositif de conversion (4),
- une ouverture numérique côté capteur de l'au moins un objectif (5) se trouve dans la plage de 0,04 à 0,1, de préférence dans la plage de 0,06 à 0,08,
- l'au moins un objectif (5) présente un diaphragme, dans lequel le diaphragme présente un rayon dans la plage de 2,5 mm à 3,5 mm, de préférence dans la plage de 2,9 à 3,3,
- l'ophtalmoscope (1) comprend précisément huit lentilles (6) et/ou précisément deux plateaux circulaires (20),
- au moins une lentille (6) est réalisée de manière asphérique,
- au moins un réticule de point lumineux (21) est agencé dans l'au moins un objectif (5), de préférence localisé de manière centrale sur un axe optique (22) de l'au moins un objectif (5), dans lequel il est de préférence prévu que l'au moins un réticule de point lumineux (21) comprenne au moins une structure diffractive (23), dans lequel l'au moins une structure diffractive (23) présente une extension dans la plage de 25 µm à 75 µm, de préférence dans la plage de 40 µm à 55 µm et/ou l'au moins un réticule de point lumineux (21) est recouvert côté capteur par un masque (24), et/ou
- le boîtier (3) comprend au moins un cylindre de bord (25), dans lequel l'au moins un cylindre de bord (25) est réalisé de manière noircie de préférence dans la zone de l'au moins un réticule de point lumineux (21).

8. Ophtalmoscope (1) selon la revendication 7, dans lequel la lumière de l'œil (2) peut être transmise dans l'ordre chronologique par une lentille d'objectif (26), le réticule de point lumineux (21), une lentille ménisque (27), une lentille d'air (14) côté objet, une lentille divergente (28) côté objet, l'obturateur, une lentille convergente (29), une lentille achromatique (30), une lentille ménisque (27) côté capteur, une lentille divergente (28) côté capteur et un verre de protection, dans lequel la lumière de l'œil (2) peut être rencontrée ensuite sur l'au moins un dispositif de conversion (4), dans lequel il est de préférence prévu que la lentille d'objectif (26) et/ou la lentille ménisque (27) côté objet et/ou la lentille achromatique (30) comprenne du verre flint et/ou la lentille convergente (29) et/ou la lentille achromatique (30) comprenne du verre crown.

9. Ophtalmoscope (1) selon l'une quelconque des revendications précédentes, dans lequel
- au moins un dispositif de retenue (31) est prévu pour une tête (32), de préférence avec un support de menton (33) et/ou un support frontal (45), dans lequel il est de préférence prévu que le dispositif de retenue (31) soit espacé de l'au moins un dispositif de conversion (4) dans la plage de 30 mm à 200 mm, de préférence dans la plage de 40 mm à 100 mm,
- l'au moins un dispositif de conversion (4) peut être amené en liaison de données avec au moins un ordinateur (34), dans lequel l'au moins un ordinateur (34) peut être amené en liaison avec une base de données,
- au moins un éclairage (35) est agencé sur le boîtier (3), dans lequel l'illumination de l'œil (2) est recouverte par le biais de l'au moins un éclairage (35) par endroits par l'au moins un objectif (5), dans lequel il est de préférence prévu que l'au moins un éclairage (35) soit configuré sous la forme d'un éclairage de lumière blanche, de préférence avec une nuance de blanc similaire à la lumière du soleil avec une température de couleur caractéristique entre 5000 K et 6000 K et/ou un indice de rendu de couleur d'au moins 95 %, et/ou un éclairage fluorescent et/ou l'éclairage fluorescent présente une plage de longueur d'onde entre 450 nm et 510 nm et/ou entre 750 nm et 780 nm,
- au moins un filtre de fluorescence (43) est agencé entre l'au moins un dispositif de conversion (4) et l'œil (2), et/ou
- l'ophtalmoscope (1) comprend au moins un écran de statut (44), dans lequel au moins une information électronique est visualisable sur l'au moins un écran de statut (44).

10. Ophtalmoscope (1) selon l'une quelconque des revendications 1 à 9 avec une lampe à fente (36).

11. Utilisation d'un ophtalmoscope (1) selon l'une quelconque des revendications 1 à 10 sur l'œil humain (2).

12. Procédé d'examen d'yeux (2) comprenant les étapes suivantes
- l'ajustement grossier d'un patient dans un dispositif de retenue (31), en particulier avec une précision inférieure à 3 mm,
- la reconnaissance d'une pupille et/ou d'un sommet d'une cornée de l'œil (2) par le biais d'un programme de traitement d'image, dans lequel il est en particulier prévu que le programme de traitement d'image présente un algorithme de mise au point automatique,
- la prise d'au moins une reproduction (16) de l'œil (2) par un ophtalmoscope (1) selon l'une quelconque des revendications 1 à 10, dans lequel l'au moins une reproduction (16) de l'œil (2) est prise sur au moins un dispositif de conversion (4).

13. Procédé selon la revendication 12, dans lequel
- des déplacements de l'œil (2) inférieurs à 3 mm sont suivis par une poursuite automatisée et/ou une ouverture d'une paupière est reconnue par le biais du programme de traitement d'image, dans lequel il est de préférence prévu que lors d'une ouverture inférieure à 12 mm, de préférence inférieure à 16 mm, une indication soit affichée sur au moins un écran de statut (44),
- l'au moins une reproduction (16) de l'œil (2) est lue et/ou enregistrée sur l'au moins un dispositif de conversion (4) dans au moins un ordinateur (34), de préférence dans une base de données sur l'au moins un ordinateur (34),
- l'au moins une reproduction (16) de l'œil (2) est évaluée et/ou analysée en ce qui concerne des caractéristiques spécifiques aux yeux,
- l'au moins une reproduction (16) de l'œil (2) est catégorisée sur au moins un ordinateur (34) de standardisation et/ou de normalisation,
- l'œil (2) présente une lentille de contact et un liquide lacrymal enrichi avec un colorant fluorescent, dans lequel le liquide lacrymal est irradié avec un éclairage (35) fluorescent, le liquide lacrymal émet de la lumière dans une plage de longueur d'onde entre 515 m et 530 nm et/ou entre 825 nm et 835 nm et une distribution de la lumière émise du liquide lacrymal est prise entre la lentille de contact et l'œil (2) sur l'au moins un dispositif de conversion (4), et/ou
- l'œil (2) comprend au moins un colorant fluorescent et une structure de vaisseau sanguin (46) et/ou une structure de vaisseau lymphatique et/ou un épithélium cornéen de l'œil (2) est imagé(e), de préférence par le biais de l'au moins un filtre de fluorescence (43), sur l'au moins un dispositif de conversion (4), et/ou est enregistré(e) sur l'au moins un ordinateur (34), dans lequel il est de préférence prévu que le colorant fluorescent comprenne de la fluorescence et/ou du vert d'indocyanine.

14. Ophtalmoscope selon l'une quelconque des revendications 1 à 10, avec un produit de programme informatique, comprenant des ordres, qui, lors de la réalisation par une unité de calcul (40), amènent celle-ci à classifier, pour l'ophtalmoscope (1) selon l'une quelconque des revendications 1 à 10 pour l'examen d'yeux (2) à partir d'une unité de mémoire (41) qui est dans une liaison de données avec l'unité de calcul (40) ou peut être amenée dans une telle liaison, au moins une reproduction (16) d'un œil (2), dans lequel une classification est générée sur la base d'une structure de vaisseau sanguin (46) et/ou d'une structure de cornée et/ou d'une structure de cicatrice et/ou d'une structure d'orbite.

15. Ophtalmoscope (1) selon la revendication 14, dans lequel, par le produit de programme informatique
- une superposition d'image avec une reconnaissance de vaisseau sanguin automatique et/ou reconnaissance de limbe peut être utilisée pour le contrôle de la classification,
- une reconnaissance de lésion semi-automatique et/ou des mesures bidirectionnelles peuvent être entreprises,
- des reproductions (16) standardisées au moins d'un iris sont enregistrées dans l'unité de mémoire (41), dans lequel il est de préférence prévu qu'en fonction d'une couleur de deux iris, une couleur d'une lentille de contact et/ou d'un iris artificiel soit sélectionnée, dans lequel la couleur de la lentille de contact et/ou l'iris artificiel est adaptée à l'un des deux iris,
- la structure de vaisseau sanguin (46) est automatiquement reconnue de préférence par le biais d'une intelligence artificielle,
- une quantification de modifications de la structure de vaisseau sanguin (46) et/ou de la structure de cornée est calculée par le biais de la superposition d'image,
- une densimétrie de pixel rouge est entreprise, dans lequel la mesure de pixel rouge intègre une part rouge entière de l'au moins une reproduction (16) et/ou calcule une part de surface de la structure de vaisseau sanguin (46) au niveau de l'au moins une reproduction (16), et/ou
- une ouverture de l'œil (2) d'au moins 12 mm, de préférence d'au moins 16 mm est reconnue et lors d'une ouverture inférieure à 12 mm, de préférence inférieure à 16 mm, une information électronique est transmise à l'au moins un écran de statut (44), et/ou le produit de programme informatique comprend une reconnaissance de lésion automatique de l'œil (2).
